(19) Europäisches Patentamt / European Patent Office / Office européen des brevets

(11) **EP 3 660 752 A1**

(12) **EUROPEAN PATENT APPLICATION**
published in accordance with Art. 153(4) EPC

(43) Date of publication:
**03.06.2020 Bulletin 2020/23**

(51) Int Cl.:
***G06N 99/00*** (2019.01)   ***G06Q 50/22*** (2018.01)

(21) Application number: **18837929.1**

(22) Date of filing: **24.07.2018**

(86) International application number:
**PCT/JP2018/027735**

(87) International publication number:
**WO 2019/022085 (31.01.2019 Gazette 2019/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2017   JP 2017143065**
**09.02.2018   JP 2018021518**
**16.05.2018   JP 2018094644**

(71) Applicant: **Axion Research Inc.**
**Tsukuba City, Ibaraki 305-0047 (JP)**

(72) Inventor: **SATO, Tomoyoshi**
**Tsukuba-shi**
**Ibaraki 305-0047 (JP)**

(74) Representative: **Körber, Martin Hans**
**Mitscherlich PartmbB**
**Patent- und Rechtsanwälte**
**Sonnenstrasse 33**
**80331 München (DE)**

(54) **ASSISTANCE SYSTEM FOR ESTIMATING INTERNAL STATE OF SYSTEM-OF-INTEREST**

(57)   A support system (10) estimates the internal state of a target system and includes: an inference module (22) configured to output a first estimated state estimated according to at least one predetermined rule, based on first test data; an AI module (21) that includes a learning module (210) configured to learn to estimate a state of the target system based on the first test data and the first estimated state, and is configured to output a second estimated state for second test data; a verification module (23) configured to verify the accuracy of the state estimated by the inference module and the state estimated by the artificial intelligence module; and a determination module (28) that switches priority rankings between the state estimated by the inference module and the state estimated by the artificial intelligence module to output, based on a verification result of the verification module.

Fig. 1

## Description

TECHNICAL FIELD

The present invention relates to a support system that estimates an internal state of a target system and includes an artificial intelligence module.

BACKGROUND ART

Japanese Laid-open Patent Publication No. 2017-91586 describes the provision of a health care server that can generate appropriate advice messages from the diet and health status of a subject for health care to increase the subject's motivation to follow a health care curriculum. The publication describes a health care server which is connected via a network to a terminal owned by the subject of health care and includes: a receiving unit that receives curriculum information, which has been selected by the health care subject, from the terminal; a storage unit that stores a behavior pattern of the health care subject; an analysis unit that analyzes continuing patterns where behavior based on the curriculum information is continuing and stopped patterns where behavior based on the curriculum information has stopped; an artificial intelligence unit that estimates physical information and the appearance of the health care subject that have been predicted for the future; a generating unit that generates the physical information and appearance; and a transmission unit that transmits as image information or text information to the terminal.

SUMMARY OF INVENTION

A system that uses artificial intelligence to analyze and test the subject (target, object) and diagnose (estimate, study, or judge) the state of the subject based on various data about the subject, and predict the future of the subject has been proposed. However, it is not easy to build-up a system that is useful for supporting actual diagnosis or the like using artificial intelligence.

One aspect of the present invention is a support system that estimates an internal state of a target system and includes a set of modules. The set of modules includes: an inference module configured to output a first estimated state of the target system that has been estimated according to at least one predetermined rule, based on first test data relating to the target system; an artificial intelligence module configured to output a second estimated state of the target system for second test data; a verification module configured to verify accuracy of the state estimated by the inference module and the state estimated by the artificial intelligence module; and a determination module that switches priority rankings of the state estimated by the inference module and the state estimated by the artificial intelligence module to output, based on a verification result of the verification module. In addition, the artificial intelligence module includes a learning module, which is configured to perform learning to estimate a state of the target system based on the first test data and the first estimated state. The target system may be a human body, may be mechatronics, such as an engine, may be a service, such as finance or investments, or may be a game.

In the initial learning stage of the learning module, the support system gives priority to the state estimated by the inference module and outputs the result as an estimation result of the support system. As the learning by the learning module progresses and the state (estimation result) estimated by the artificial intelligence module exceeds the accuracy of the state (estimation result) estimated by the inference engine, the state estimated by the artificial intelligence module is outputted with priority as the estimation result of the support system. By comparing the estimation result of the artificial intelligence module which includes the learning module with advanced learning with the result estimated based on a predetermined rule by the inference module, it becomes possible to verify one estimation result using the other estimation result. For example, for the cases where the rules included in the inference module struggle to cope, the artificial intelligence module that includes a learning module with advanced learning may estimate the internal state with relatively high accuracy, and such estimation result may be verified using the estimation result of the inference module. Even if the applicable range of the rules included in the inference module is limited, the estimable range of the artificial intelligence module may be expanded through learning.

The set of modules may further include a replica generating module configured to generate replicas of the test data of the target system for learning by the learning module. Learning by the learning module may be promoted by processing a large number of replicas. The set of modules may further include an optimization module configured to provide an optimization policy for the replicas to the replica generating module based on the accuracy of the state estimated by the artificial intelligence module that has been verified by the verification module. Learning can be promoted for results where the accuracy of the state estimated by the artificial intelligence module is low. In addition, learning by the learning module can be promoted by mass-producing replicas for phenomena where there is insufficient real data. The learning performed using replicas may be supervised learning in the same way as real data in which the inference module is involved, or may be unsupervised learning where the inference module is not involved, or may be reinforcement learning that has accuracy, such as ambiguity at the verification module, as a reward.

The replica generating module may include a module that generates replicas reflecting actual distributions of a plurality of parameter values included in the test data of the target system. The learning module may automatically learn features indicating the internal state of the target system using a large number of replicas reflecting the actual distributions as training data. The replica generating module may include a module that generates internal replicas indicating the internal states of the target system and replicas of test data reversely estimated from ranges of change in the internal replicas. The learning module may perform supervised learning with replicas of the test data with the internal replicas as labels.

The set of modules may further include an input processing module configured to pre-process test data of the target system according to a predetermined rule to convert to test data to be inputted into the inference module and the artificial intelligence module. By performing statistical processing or correlation processing on the test data in advance according to a predetermined rule, there is a possibility that the logic or inference in the artificial intelligence module may be easily traced or the basis of such logic or inference may be easily estimated. As one example, the input processing module may include a module that generates a map indicating correlation between parameters included in the test data. In a complex system, there is the possibility of there being a large number of correlations between the parameters, but by mapping in two dimensions or three dimensions, such correlations may become subjects (objects) for learning using image recognition.

The verification module may include a module that determines the ambiguity of the state estimated by the inference module or the artificial intelligence module, and a module that acquires additional information from outside according to the ambiguity. The additional information may include at least one of feedback from a diagnosis expert on the target system, a follow-up observation, and results of additional tests on the target system. It becomes possible to improve the ambiguity of an estimation result that has high ambiguity (a large margin of ambiguity), to improve the ambiguity or noncommittalness of the estimation result of the inference module, or to teach the learning module using an estimation result with little ambiguity.

The set of modules may further include a module that outputs the estimated state selected by the determination module as diagnosis information for the target system. The output method of the diagnosis information may change depending on the side (user) receiving the diagnosis information. As one example, the diagnosis information of the target system may be expressed as symptoms of a digital avatar, the social type of the user may be analyzed in advance, and the diagnosis information may be outputted according to a method to which that social type is receptive or which appears familiar to that social type.

The support system may include a memory storing the set of modules as a program (program product); and a processor that loads and executes the program stored in the memory.

Another aspect of the present invention is a cloud system where at least one support system disclosed above is connected to a terminal of a diagnosis expert on the target system via the cloud, and the output of the at least one support system is referred to via the cloud (the Internet or another computer network). In a system in which the support system, the terminal of the diagnosis expert on the target system, and an institution capable of follow-up observations of the target system are connected via the cloud, the verification module may order advice from a diagnosis expert or a follow-up observation via the cloud.

Another aspect of the present invention is a program (program product) including instructions for causing a computer to function as the support system described above. The program may be provided by being recorded on an appropriate recording medium.

Yet another aspect of the present invention is a control method of a support system that estimates the internal state of a target system. The support system includes: an inference module configured to output a first estimated state of the target system that has been estimated according to at least one predetermined rule, based on first test data relating to the target system; an artificial intelligence module that includes a learning module, which is configured to perform learning to estimate a state of the target system based on the first test data and the first estimated state, and is configured to output a second estimated state of the target system for second test data; and a verification module configured to verify accuracy of the state estimated by the inference module and the state estimated by the artificial intelligence module. The control method includes steps of: outputting the state estimated by the inference module with priority over the state estimated by the artificial intelligence module; and outputting the state estimated by the artificial intelligence module with priority over the state estimated by the inference module.

The support system may also include a replica generating module configured to generate replicas of the test data of the target system for learning by the learning module, and the control method may further include a step of providing the replica generating module with an optimization policy for replicas based on the accuracy of the state estimated by the artificial intelligence module which has been verified by the verification module.

BRIEF DESCRIPTION OF DRAWINGS

[FIG. 1] FIG. 1 is a block diagram depicting one example of a diagnosis support system.
[FIG. 2] FIG. 2 is a block diagram depicting one example of a correlation processing module.
[FIG. 3] FIG. 3 is a diagram depicting one example of a heat map.
[FIG. 4] FIG. 4 is a diagram depicting an enlargement of part of a heat map.
[FIG. 5] FIG. 5 is a diagram depicting another example of a heat map.

[FIG. 6] FIG. 6 is a diagram depicting yet another example of a heat map.

[FIG. 7] FIG. 7 is a diagram comparing a plurality of heat maps.

[FIG. 8] FIG. 8 is a diagram depicting yet another example of a heat map.

[FIG. 9] FIG. 9 is a diagram depicting one example of a vector map.

[FIG. 10] FIG. 10 is a diagram depicting how a heat map changes together with a state.

[FIG. 11] FIG. 11 is a diagram depicting how correlation changes together with the state.

[FIG. 12] FIG. 12 is a diagram depicting an example of an individual matrix.

[FIG. 13] FIG. 13 is a block diagram depicting one example of a learning system.

[FIG. 14] FIG. 14 is a block diagram depicting one example of an inference engine.

[FIG. 15] FIG. 15 is a block diagram depicting one example of a verification process.

[FIG. 16] FIG. 16 is a block diagram depicting one example of a verification engine.

[FIG. 17] FIG. 17 is a diagram depicting one example of a control process of a support engine.

[FIG. 18] FIG. 18 is a block diagram depicting one example of a final determination/deciding module.

[FIG. 19] FIG. 19 is a block diagram depicting one example of functions of an output processing module.

[FIG. 20] FIG. 20 is a block diagram depicting one example of a replica generating module.

[FIG. 21] FIG. 21 is a block diagram depicting one example of a cloud service.

[FIG. 22] FIG. 22 is a block diagram depicting one example of functions of the cloud service.

[FIG. 23] FIG. 23 is a block diagram depicting one example of expansion of the cloud service.

DESCRIPTION OF EMBODIMENTS

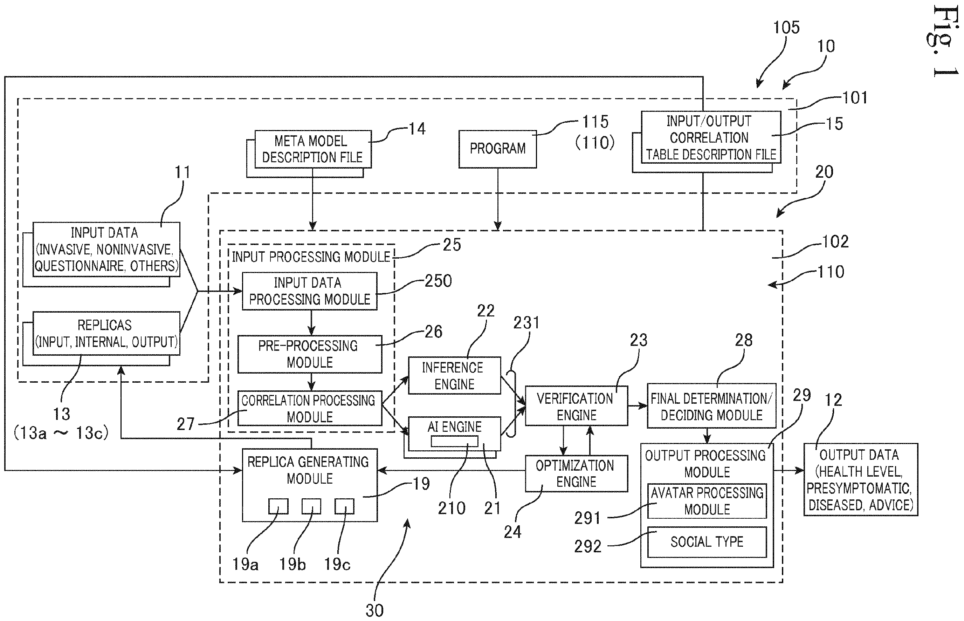

FIG. 1 depicts an example of a support system according to the present invention. This support system 10 is an example of a diagnosis support system (diagnosis assistance system) that has a person (human body, human) as a subject (target) of diagnosis (subject, target system). This support system (called as "Axion system") 10 has a hybrid engine (diagnosis support engine, called as "Axion engine", or "AXiR engine") 20 including: an artificial intelligence (AI) engine (AI module) 21 including a learning module 210; an inference engine (inference module) 22 including a function as an expert system of a doctor who examines (diagnoses) a human as the subject (target system); and an educational system (learning system) 30 that generates replicas 13 as teaching data for teaching the learning module 210 of the artificial engine 21, based on the estimation results of the AI engine and the inference engine 22 for input data 11 that is test data related to the target system.

The support system 10 includes a memory 101, which stores various data, programs (program products) 115, and the like, and a processor 102, which executes a set 110 of modules stored as a program 115. The program (program product) 115 includes a plurality of instructions that cause a computer 105 including the memory 101 and the processor 102 to function as the support engine 20 and the support system 10.

The support engine 20 is provided as a unit of set 110 of a plurality of modules that can be executed by the processor 102. In the following description, the set 110 of modules is described as the support engine 20. The processor 102 may be a general-purpose processor, an ASIC equipped with a special-purpose circuit, or a reconfigurable processor such as an FPGA or a DRP.

1. System Overview

The support engine 20 is a diagnosis support engine 20 that has a human body as a target system whose internal state is to be estimated. The diagnosis support engine 20 includes: an inference module (inference engine) 22 configured so as to output a first estimated state of a target system, which is estimated in accordance with at least one predetermined rule based on first test data (first input data) relating to the human body that is the target system; and an artificial intelligence module (AI module, AI engine) 21 including a learning module 210 configured so as to learn to estimate the state of the target system based on the first test data and the first estimated state. The AI engine 21 may be configured to output, when learning by the learning module 210 has progressed, a second estimated state that may differ to the state estimated by the inference engine 22 for the second test data. The diagnosis support engine 20 further includes: a verification module (verification engine) 23, which is configured to verify the accuracy of the state estimated by the inference engine 22 and the state estimated by the AI engine 20; and a determination module (final determination/deciding module) 28 that switches the priority rankings of the state estimated by the inference engine 22 and the state estimated by the AI engine 21 to output, based on the verification result of the verification engine 23.

The support engine 20 further includes: a replica generating module 19, which is configured to generate the replicas 13 of the test data 11 of the target system for learning by the learning module 210; and an optimization module (optimization engine) 24, which is configured to provide an optimization policy for the replicas 13 to the replica generating module 19, based on the accuracy of the state estimated by the AI engine 20 that has been verified by the verification engine 23. The support engine 20 further includes an input processing module 25 configured to pre-process the test data 11 of the target system according to predetermined rules to convert to test data to be inputted into the inference module 22 and the AI module 21. The support engine 20 further includes a module 29 that outputs the estimated state that has been selected by the determination module 28 as diagnosis information (output data) 12 for the target system. The output processing module 29 may include an avatar processing module 291 that expresses and outputs the diagnosis information for the target system as symptoms of a digital avatar and a social type analysis module 292 that analyzes the user's social type in advance and outputs according to a method to which that social type is receptive or which appears familiar to that social type.

In this support engine 20, first, the learning module 210 of the AI engine 21 performs learning with results produced by analysis or inference of the input data (test data) 11 according to predetermined rules implemented in the inference engine 22 as teaching data. In addition to this, the learning module 210 is trained by the input replicas 13 generated in response to feedback from the verification engine 23 and the optimization engine 24 to realize automatic or autonomous improvement in diagnostic accuracy. Since the support engine 20 is a hybrid implementation of two engines, namely the inference engine 22 and the AI engine 21, after the AI engine 21 has been trained, it is possible for the support engine 20, from the viewpoints of both the inference engine 22, which is an expert medical system, and the AI engine 21, to make inferences and estimations of the activity state (such as "healthy", "unhealthy", "presymptomatic ("Mibyou" in Japanese)"), dysfunctions and failures (disease levels), deterioration, tumors, carcinogenesis of internal elements of the body from the input data 11 that includes non-invasive and/or invasive measurement data, and to provide advice, valuable health information, and perspectives (possibilities and risks) that tend to be overlooked.

In other words, the diagnosis support engine 20 is characterized by the self-learning function of the AI engine 21 and two engines, that is, the verification engine 23, and the optimization engine 24, functioning to support this self-learning, with the object of improving the accuracy of predictions and inference. In particular, although the inference engine 22 and the AI engine 21 function as a hybrid type, the diagnosis support engine 20 is characterized in that switching to the AI engine 21 is performed when the prediction and inference accuracy of the AI engine 21 continuously exceeds that of the inference engine 22. The system is constructed so that a replica generating module 19 is present on the outside so as to allowing functioning even with a small amount of input data, with the variation in replica generation changing according to instructions from the optimization engine 24.

1.1 Overview of AI Engine

The AI engine 21 to be implemented is based on deep learning, and the AI engine 21 may include a plurality of learning modules 210 of different types. The learning system 30 uses three engines, the inference engine 22, the verification engine 23, and the optimization engine 24 so that the learning module 210 of the AI engine 21 can function even with a small amount of input data 11 (to enable autonomous training), and supports growth of the AI engine 21 by supporting the AI engine 21 and acting as part of the self-learning function. The three engines 22 to 24 of the learning system 30 also have a function of improving the prediction accuracy/inference accuracy of the diagnosis support engine 20 and the AI engine 21. That is, the learning system 30 functions as a type of feedback circuit for improving accuracy.

According to this learning system 30, the AI engine 21 will exceed the prediction accuracy of the inference engine 22 in stages. The win ratio between the AI engine 21 and the inference engine 22 is determined by the verification engine 23 and the final determination/deciding module 28 that performs a final determination downstream of the verification engine 23, and in an initial stage, the engine 23 and the module 28 give priority to the diagnosis results ("inferences" or "predictions") of the inference engine 22. After that, as one example, if the AI engine 21 exceeds a win ratio of 80% on average for three months or the win ratio continuously exceeds 90%, the verification engine 23 and the final determination/deciding module 28 give priority to the diagnosis results of the AI engine 21, the inference engine 22 is placed on the backup side, and the inferences and predictions of the inference engine 22 are not used for the time being. However, the verification engine 23 and the optimization engine 24 continue functioning and supporting the system, even when the main operation is performed by the AI engine 21.

Accordingly, the function, nature, performance, ability and the like of the diagnosis support engine 20 are influenced by the three engines included in the learning system 30, that is, the inference engine 22, the verification engine 23, and the optimization engine 24. These engines 22 to 24 are fundamentally based on the concept of a meta model. In other words, in the system to be diagnosed, a complex system such as the human body for which it is necessary to predict abnormalities and failures with high accuracy, there are system elements and components that construct or assemble the system, and the functioning of these elements or components serve as the basis for predictions of the states mentioned above and enable the inputs and outputs of the system to be defined. Here, it is possible to assume that a favorable, unfavorable, defective, or broken down state of the subject (target system) of diagnosis has occurred from problems with the internal elements or components, regardless of the extent of the state, and use a design based on the above for predicting the causes or problems from measurement data and/or observation data, giving advice on countermeasures, and providing information that is necessary and/or information that is expected for that situation .

This configuration uses a meta model description file 14, in which the functions of these engines 22, 23, and 24 are written, to describe the models of definitions and/or correlation information, input information, statistical information, and evaluation information in the memory 101 located outside the diagnosis support engine 20, that is, in an independent system and/or on the cloud to the greatest extent possible, therefore this configuration may be adapted to a plurality of systems and applications by changing the written content of the description information. Accordingly, by changing the input data 11 and the meta model description file 14, the support engine 20 can have a different role, range of protection, and response. In addition, even in the same target field of application, by using specialized input data 11, it is possible to create (train) support engines 20 that have different specialties and personalities. Although the explanation given below has a premise of a meta model description file 14 based on input data (test data) 11 in different fields, such as health measurements, breast cancer, pancreatic cancer, kidney cancer, lung cancer, stomach cancer, liver cancer, colon cancer, and the like in the health care field, the field in which the diagnosis support engine 20 can be applied is not limited to health care and may be an industrial field, such as gas turbine engines and factory automation, a commercial or service field such as finance, or an entertainment field such as (video) games.

1.2 Overview of Inference Engine

In more detail, the inference engine 22, which is one of the core elements forming the diagnosis support engine 20, performs inference from the input data 11 relating to tests (examinations, inspections), including measurement data, medical interview data, and the like, estimates the internal state of the human body that is the subject of diagnosis, and estimates the presence or absence of abnormalities, such as diseases and cancer. The inference engine 22 is an expert system that includes one or a plurality of rules that have been generated or derived based on information from medical personnel, such as doctors, and information, experiences, and the like relating to past diseases. The input data 11 includes a plurality of elements, such as medical interviews, blood, urine, statistics, microRNA, and heredity, and the engine 22 has a function of narrowing down the inferred states of the human body by suggesting several candidates based on such elements. When the prediction/estimation accuracy of the AI engine 21 is not sufficient, the inference engine 22 takes the lead for the time being and executes the diagnosis support service of the diagnosis support engine 20. The final determination/deciding module 28 decides the timing of switching of the priority of the estimation results of the AI engine 21 and the inference engine 22 in accordance with the meta model description file 14. Even if the base or principal of diagnosis support is switched to the AI engine 21, the inference engine 22 continues to function in the background and the inference result is always compared with the estimation result of the AI engine 21 to achieve a function of constantly verifying the estimation result of the AI engine 21.

The inference engine 22 of the diagnosis support engine 20 estimates the internal state of the system to be diagnosed using the test data 11 that are bases for estimations about internal parts (elements) that are in a direct relationship or a causal relationship with the favorable, unfavorable, disordered, failing, or damaged state of the complex system to be diagnosed, and about their relationships, activity levels, recovery function, immune system, adjustment mechanism, and deterioration factors. Correlation between internal parts (elements) that are in a direct relationship or a causal relationship with the favorable, unfavorable, disordered, failing, or damaged state of the complex system to be diagnosed and their relationships, activity levels, recovery function, immune system, adjustment mechanism, and deterioration factors and test parameters, numerical values, and the like included in the test data 11 may be given by the meta model description file 14. One of the rules implemented in the estimation engine (inference engine) 22 may include: estimating internal replicas of the target of diagnosis from the input data 11 as the first-order predictions; if the first-order predictions include reliable predictions within a certain range, estimating the reliable predictions as the second-order predictions; and re-estimating the input data based on the second-order predictions. From the second-order predictions, it is possible to estimate the time and damage of the favorable, unfavorable, disordered, failing, or damaged (boundary) state of the system that are of concern in the future as the third-order predictions.

When determining the output data 12 that is the final inference result (diagnosis information) of the diagnosis support engine 20, an internal state (internal replica) among a variation of internal replicas inferred by the input data (which may include measurement data, a medical questionnaire, or the like) 11, with an input replica regenerated from the internal state (internal replica) and having the smallest difference (variance) with the input data and measurement data, may be given as a leading candidate for the inference result.

The inference engine 22 may include a rule for estimating an estimation result from an input/output correlation table/description file 15 in which the correlation with the input data 11 is described. If it is difficult to narrow down the states that can be estimated, a rule that improves the estimation accuracy through the verification engine 23 making a decision and additional tests being performed to obtain new input data 11 and medical interview data may be provided. This rule may be used in the optimization engine 24, which contributes to the improvement of accuracy of prediction and estimation. The input/output correlation table/description file 15 needs to be periodically reviewed and updated for the purpose of ensuring the improvement in accuracy. The correlation table information may include information indicating the relationship between input data or medical interview information and internal parts (organs, blood vessels, bones, spinal cord, and the like) of the human body.

1.3 Overview of Verification Engine

The verification engine 23 may verify the accuracy (correctness, truthfulness) of the predicted or estimated state against the estimated internal state (internal replica) included in the estimation result 231 of the inference engine 22 or the AI engine 21 in accordance with the evaluation formulas (such as algorithms, formulas, least-square methods, or the like) in the meta model description file 14. The accuracy (truthfulness) of the estimation result may be verified for a regenerated input replica that is regenerated from the internal replica. When the accuracy of the estimation result 231 is insufficient and there is large ambiguity, the verification engine 23 may indicate a verification result that requests a testing by a medical doctor (MD) or additional testing, or continuous testing in units of every two weeks. In addition, for the cases that ambiguity remains in the determination, a determination cannot be made, or the narrowing down is insufficient, the verification engine 23 may indicate a verification result that sets a continuous testing (verification) flag for the estimation result so that follow-up testing continues. That is, the verification engine 23 may include a function operable, when it has been determined that the evaluation of the estimation results 231 of the AI engine 21 and/or the inference engine 22 cannot be established at this time, to post the estimation results 231 on a timeline (in a time capsule) and perform verification after a certain time has passed (a follow-up observation function). These options may be included in the meta model description file 14.

The verification engine 23 may include a function for attempting to obtain further information when it has been determined that the input data 11 is insufficient for evaluation of the estimation result 231 of the AI engine 21 and/or the inference engine 22. The input data (test data) 11 may include as the measurement data for determining the health level, for example, but not limited to, blood tests, urine tests, breath/skin gas measurements, measured values of body temperature, visceral fat, subcutaneous fat, muscle mass, body fat percentage, estimated bone mass, blood pressure, blood flow, heart rate, autonomic nervous excitement, and the like, image diagnosis results, and information on microRNA, ncRNA, and/or DNA. Such information and information produced by a medical questionnaire may be included in the input data 11 as test data. The verification engine 23 may include a function for proposing additional tests. As one example, the verification engine 23 may include a function that links to disease candidates that can be imagined from the estimation results 231 of the AI engine 21 and/or the inference engine 22 and orders additional tests to confirm the potential of such disease candidates. When this configuration is used, the verification engine 23 may include a function that receives proposals from a medical doctor and gives priority to these proposals. As additional measurements to be used for verification, a method that sequentially adds measurements may be adopted.

The verification engine 23 may also include a function operable when it is difficult to immediately perform these additional tests for any reason or it is difficult to perform additional measurements immediately, to post a request of the additional tests s to a time capsule, to measure again after several months, half a year, one year, or more have passed as with the determination treated as "pending", and then feed back the difference between the measurements to the prediction or inference that has remaining doubts to update. This function can be included as part of a follow-up observation function, and when it is necessary to continue follow-up observations, it is possible as one example to use a method where a detailed examination is performed in units of every two weeks, repeat several times for example, and make a differential observation from the differences between the examinations to judge whether the subject is becoming healthier or is deteriorating and becoming more diseased.

1.4 Overview of Optimization Engine

The optimization engine 24 gives instructions on the policy for optimizing the replicas 13 so that the prediction and estimation accuracy (truthfulness) of the AI engine 21 is fundamentally improved. To do so, the optimization engine 24 may adjust initial parameters in the description file 14 and may instruct changes to the algorithm used to increase the inference accuracy and prediction accuracy. As one example, when there is the suspicion of an infection in a certain workplace, region, or organization, the optimization engine 24 may issue instructions on a policy to generate the replicas 13 so as to increase the priority of predictions and estimates related to this situation. If many manufacturing defects or variations have been observed for specific parts of a system to be diagnosed, the optimization engine 24 may issue instructions to give greater weighting to parameters related to the estimation of these defects so as to raise the level for generating replicas that are effective in learning to estimate such defects.

2. Replica

2.1 Overview of Replica Generating Module

Although the expression "deep learning" gives a mistaken impression that anything can be done, it is ultimately necessary to move closer to perfection by performing learning on a huge number of input data. In other words, from the viewpoint of conventional debugging and system construction, deep learning should be considered to be something completely different. In particular, when a system is complex, there is no single function, and there is the burden of having to perform certain procedures to make corrections and obtain the expected results.

In this system, the replica generating module 19 acts to facilitate this process as much as possible through control on the input side. For example, to realize an improvement in a short time by preparing input patterns in advance as a pilot and measuring accuracy by learning performed on input data, it may be possible to intentionally raise performance in stages by measuring accuracy of prediction using a method which prepares, for a given timing to the next input timing, a plurality of patterns and compares a plurality of results of the patterns to compere (judge) what input replicas give favorable results. The prediction and inference accuracy may be improved using a method that compares the inference result of the inference engine 22 and the inference result of the AI engine 21, evaluates the result of learning from patterns with different inference results, and changes the input replicas. The replica generating module 19 can provide a self-learning model in which rules are established for the generation pattern of replicas used for self-learning and feeding back is performed while the system is learning by itself.

In classical system engineering, by using a method where a system is constructed by combining modules that have a single function, it is possible to guarantee that such system will be secure and reliable. However, this method is not very flexible, but if modules have a single function, during debugging it is easy to understand the cause of problems and easy to provide backups, which makes it easy to identify and handle future failures and other troubles.

The replica generating module 19 may be provided as an external tool. The replica generating module 19 may be incorporated in the support engine 20, which makes it possible to control replica generation from the input processing side on the inside via instructions given by the optimization engine 24. The AI engine 21 is capable of learning the basic features through learning performed fundamentally on the input data 11. By performing self-learning using a large number of replicas 13, it is possible for the AI engine 21 to evolve, including the learning of features that are not imagined (predicted, foreseen) by the inference engine 22.

Together with the inference engine 22, the verification engine 23, and the optimization engine 24, the replica generating module 19 is one of the important elements constructing the learning system 30. In a state where a sufficient amount of real data is not available, the replica generating module 19 has a role as a module that makes up for the insufficiency in real data by providing input data for accelerating the learning by the AI engine 21 and also assists the engines used for inference, verification, and optimization. In particular, the replica generating module 19 may be a tool for generating states (replicas) of internal elements that are used for comparisons and clearly indicate errors, defects, inconsistencies, malfunctions, damage, and breakages so as to facilitate verification. To check the accuracy of inferences through further comparisons of the active states of functions, the replica generating module 19 may include a method of regenerating input replicas from internal replicas 13b that have been generated. When doing so, in addition to the variations of input replicas 13a that were generated first, by estimating variations of internal replicas 13b generated from the input data 11, regenerated input replicas 13c can be conceived or regenerated based on such estimated variations. Compared to the real data (input data) 11, there is a higher number of high potential replicas that exist as variations of the internal replicas 13b, which makes it possible to improve the prediction/estimation accuracy of the AI engine.

The replica generating module 19 may include a function as a tool that efficiently promotes machine learning by the AI engine 21. For example, for a case where the human body is the subject of diagnosis, to improve the determination and accuracy of boundary regions that are classified as diseased/morbid or healthy/presymptomatic, the replica generating module 19 may be equipped with a function that automatically generates replicas (input replicas) 13a with a larger number of variations statistically based on a plurality of measurement data.

At present, testing costs 50,000 to 60,000 yen per person for a case where a blood test, a urine tests, and/or a CT, MRI, X-ray, or the like are assumed. On the other hand, although data on tens of thousands to millions of subjects would be ideal for bringing the AI engine 21 closer to perfection, it is not easy to secure a system and a budget capable of collecting such data in a short time. The replica generating module 19 is capable of automatically increasing the data required for learning by the AI engine 21, including variations, based on a plurality of measurement data (the test data) 11. This makes the replica generating module 19 an effective tool for promoting self-learning by the AI engine 21 in a short period of time based on a relatively low number of data sets. Also, when the number of cases is small and the number of test data 11 that can be actually measured is small, the replica generating module 19 functions as an ideal path that uses the replicas 13 to feed back self-learning results of the AI engine 21 and thereby improve the estimation accuracy of the AI engine 21.

The replica generating module 19 may generate replicas on its own. The replica generating module 19 may also be linked to the generation of internal replicas in the estimation process by the inference engine 22, and the result may be used for generation of replicas for self-learning by the AI engine 21. The replicas to be generated may include three types, input data replicas (input replicas) 13a, internal replicas 13b, and regenerated input replicas 13c. The replica generating module 19 may include an input replica generating module 19a, an internal replica generating module 19b, and a regenerated input replica generating module 19c. The input replica generating module 19a may be a module that generates the input replicas 13a which reflect the actual distribution of the values of a plurality of parameters included in the test data 11 of the target system. The internal replica generating module 19b may be a module that generates internal replicas 19b indicating the internal states of the target system. The regenerated input replica generating module 19c may be a module that generates replicas (regenerated input replicas) 13c of the test data 11 that have been reversely estimated from a range or ranges of change in the internal replicas 13b.

2.2 Input Data Replicas

The input data replicas (input replicas) 13a may be data that are generated from a probability model based on statistical data and/or real data, and are used in place of the actual test data (input data) 11. The input data 11 for health care could be a probability model specified from mean values and variance that may include a blood test, a urine test, breath/skin gas measurements, measured values of body temperature, visceral fat, subcutaneous fat, muscle mass, body fat percentage, estimated bone mass, blood pressure, blood flow, heart rate, autonomic excitability and the like, image diagnosis results, microRNA, ncRNA, DNA or the like, and medical questionnaires.

The input replica generating module 19a is a module that generates, based on statistical data and real data, data (input replicas) 13a that are used in place of actual measurement data. The replicas 13a of the input data may be generated based on a probabilistic model that assumes two main models (a simple model and a hierarchical model) for biological data. Examples of a probability distribution include a simple distribution, such as a uniform distribution, and a normal distribution.

An uncorrelated model (also referred to as "a simple model") is a model where there is no correlation between data. This is generated based on a model of a super healthy body called a "super normal". The elements Yi of the replicas follow a normal distribution expressed by Equation (1) below

$$Yi=N(\mu i,\sigma i2) \ \dots \ (1)$$

Here, $\mu i$ and $\sigma i2$ are constants, and by setting these constants for each element, respective probability models are determined.

A correlated model (also referred to as a "hierarchical model") is a case where there is correlation between data, and is a model that mainly assumes a diseased body. In this case, $\mu i$ and $\sigma i2$ are not constants and instead are values calculated in association with other elements. This means that if a hierarchical model calculated from the values of other elements is introduced into the mean $\mu i$ and variance $\sigma i2$ of the elements, it will be possible to reflect the correlation between that elements in a statistical model. When a variable that is a certain factor (explanatory variable) is x, and mean values Y1 and Y2 of other elements increase or decrease due to x, it is possible to find out correlated elements by expressing them with the following equation (2). In this case, x is designated and a1, a2, b1, and b2 are defined as constants.

$$\mu1=a1x+b1$$
$$\mu2=a2x+b2 \ \dots \ (2)$$

It is also possible to output x itself as an element of replicas. It is also possible to designate the variance value in the same way.

2.3 Internal Replicas

The internal replicas 13b are data obtained by estimating the states of internal components, that is, the states of the subject of diagnosis, with respect to the input data 11. The fundamental role of the internal replicas 13b are to serve as teacher data for the input replicas 13a. The engine 20 may generate the internal replicas 13b that have been estimated from the input data 11 based on inferences by the inference engine 22 and/or inferences by the AI engine 21. Internal components in the field of health care are mainly elements or parts of the human body, and are composed of organs, blood vessels, bones, the spinal cord, and others. The internal replicas 13b may be indices in which the activity levels of these components are indicated by numerical values. Information regarding these components may be provided by the input/output correlation table 15.

In one specific example, internal replicas 13b may relate to the measurement (test) data described below included in the input data 11, use the states of organs that are correlated as indices of activity rates or the like, and be the result in the ranges of generating that can be taken by these index values using a probability model. The internal replica generating module 19b generates internal replicas 13b for measurement data (including medical interviews) using the input/output correlation table 15 and instructions from the optimization engine 24. If the states of organs are defined at a stage where the input replicas 13a are generated for learning purposes, it is possible to treat the set of the input replicas 13a and the states of the organs as supervised data and use this as data for learning by the AI engine 21 and/or as data for use in evaluation without using the generation module 19b. Since measurement data (input data) 11 that includes a doctor's findings also forms a set of measurement data and the findings for organs, it can fundamentally be handled in the same way as supervised data. On the other hand, in order to infer the internal state from input data (measurement data) 11 that does not include a doctor's findings, it will be necessary every time to refer to the input/output correlation table 15 and generate, for the measurement data 11, variations of the internal replicas 13b that have potential. After this, regenerated input replicas 13c generated from variations of the internal replicas 13b are compared with the input data 11, and the sets of the optimal internal replicas 13b and the regenerated input replicas 13c are used as supervised data.

According to the input/output correlation table 15 and instructions from the optimization engine 24, the internal replica generating module 19b may generate internal replicas 13b based on the input replicas 13a generated as phase# 1 from the measurement data (including medical interviews) 11. In phase #1, input replicas 13a may be generated with a limited degree of freedom based on the input data (measurement data, medical interviews, and the like) 11, and internal replicas 13b may be generated on a one-to-one basis for the input replicas 13a.

As will be described later, the input/output correlation table 15 is a file that defines the correlation between the input data (test data) 11 and indices of internal states (such as the activity rates of respective parts). For measurement data where a normal range and an abnormal range are statistically understood, a statistical model may be defined where related internal states are used as indices, the normal range is expressed as "100" and abnormal ranges are assigned values that decrease in steps according to the degree of the abnormality. For disease data, indices for internal states may be defined based on the judgments of doctors for measurement data. For medical interview data, indices may be defined for interview results in accordance with internal components linked in advance at the time of the questioning and indices on the states of such components. This table 15 may be used in the reproduced input replica generating module 19c.

The internal replica generating module 19b may generate the internal replicas 13b as multi-stage replicas. As one example, first-order internal replicas may be generated from the input data 11 and stochastic predictions. Source data used here are the input data (excluding measurement data and medical interviews) 11 or input replicas 13a. When generating first-order internal replicas, correlation between one or more input data 11 may be used as a reference (weak correlation). When generating first-order internal replicas, a configuration that allows unknown elements may be used.

Second-order internal replicas may be replicas that are generated from correlations of source data and diseases. The source data used here may be are the first-order internal replicas, and may be generated from the correlations of input data and a correlation table (strong correlation: disease, illness, medical history, genetics, or the like). The generation pattern may be changed according to instructions from the optimization engine 24.

Third-order internal replicas may be replicas generated by filtering/narrowing according to medical interviews. Source data used here may be the second-order internal replicas and interview data. The third-order internal replicas may recheck the second-order internal replicas from the interview data and amend, correct, or exclude data. These processes may be executed according to instructions from the optimization engine 24.

Fourth-order internal replicas may be replicas generated with consideration to the response to spread of flu (influenza) or other infections. Source data used here may be the third-order internal replicas and a situation and/or information specified by the cloud service (P-HARP), described later. The fourth-order internal replicas recheck the third-order internal replicas according to instructions from P-HARP, and amend, correct, and exclude data. These processes may be executed according to instructions from the optimization engine 24.

Fifth-order internal replicas may be generated with consideration to response to a pandemic. Source data used here may be the fourth-order internal replica and a situation and/or information specified by P-HARP. The fifth-order internal replicas recheck the fourth-order internal replicas according to instructions from P-HARP, and amend, correct, and exclude data.

These processes may be executed according to instructions from the optimization engine 24.

2.4 Regenerated Input Replicas

The regenerated input replicas 13c are data (replicas) obtained by reading the input/output correlation table/description file 15 in the opposite direction and estimating the input data 11 from internal replicas 13b. The replicas 13c are fundamentally the same type as the input data 11, but the generated data may be only data with correlation to internal elements where an abnormality or abnormalities have been detected. By comparing reproduced input replicas 13c with the actual input data 11 and specifying an internal replica 13b, out of the variations, that is closest to the input data 11 from an evaluation function, the AI engine 21 may learn to predict and infer the internal state.

Although input replicas 13a are generated according to a distribution based on a group of healthy persons, diseased people, or "presymptomatic" people who exist between the healthy and diseased states, regenerated input replicas 13c differ by being based on the activity rates of organs. In other words, regenerated input replicas 13c may be data where the range that can be taken by the test values are generated from the activity rates using a probability model.

The regenerated input replica generating module 19c may be a module that reconstructs (rearranges, reconfigures) the input data 11 (i.e., generates a regenerated input replica) by reversely estimating the range of changes in the input data 11 with respect to the variations of the internal replicas 13b that have been generated. By generating the internal replicas 13b separately to the input replicas 13a generated by a statistical method and generating the input replicas (the regenerated replicas) 13c again from these internal replicas 13b, it is possible to perform both an operation that statistically expands the possibility of replicas and generation of replicas that indicate factors (states) that have been narrowed down from this expanded possibility. It is believed that this will make it possible to improve the estimation accuracy of the AI engine 21.

Generating the internal replicas 13b and the regenerated replicas 13c leads to the possibility of directly checking the components inside the system to be diagnosed, and contributes to improvements in the verification accuracy. It is possible to generate replicas that use data correlation that characterizes the components of the human body to be diagnosed and diseases and healthy states, and therefore possible to produce replicas that reflect one or more disease models measured after detailed examinations and/or after diseases have been confirmed better than the initial input replicas 13a generated by statistical processing. This is considered effective for having the learning effect of the AI engine 21 narrow down (targeting) to diseases.

The reproduction input replica generating module 19c has the same configuration as the input replica generating module 19a and as necessary may have enhanced functioning so as to be capable in principle of generating the necessary reproduction input replicas 13c by merely changing the specification of the model file. The conditions for generating reproduction input replicas 13c may be described in the input/output control table 15 in the same way as the internal replicas 13b. The input/output control table 15 may be used by reading in the opposite direction to the internal replicas 13b. The meta model description file 14 may be a definition file that is necessary along with the input/output correlation table 15 for generating reproduction input replicas 13c.

The reproduction input replica generating module 19c may obtain correlation information from the input/output correlation table 15 and the meta model 14 and reproduce the input replicas in multiple orders. Using the reproduction input replicas 13c, which are generated from variations of the internal replicas 13b by reading the input/output correlation table 15 in the opposite direction when internal replicas 13b are generated, the verification engine 23 may calculates difference values (dispersion) relative to the measurement data 11 and specifies active states, operating states, and/or defective states (disorder, fatigue, deterioration) of internal parts of the human body from the result.

First-order replica regeneration may be performed from internal replicas 13b by statistical prediction. The reproduction input replicas 13c may be generated by probability models according to the states (activity rates) of organs based on the statistical data in a case where there is no correlation within the test data. Second-order replica regeneration may be generated from correlations of source data and/or diseases. As a more complicated case than first-order replica regeneration, reproduction input replicas may be generated according to one or more probabilistic models in accordance with the states (activity rates) of organs based on statistical data that imagines a case of a specific disease where there is correlation within the test data. Third-order replica regeneration may be performed by filtering/narrowing using medical interviews. Depending on the states (activity rates) of organs, medical interview data may be generated as reproduction input replicas using a probability model. Fourth-order replica regeneration may be generated in response to the spread of influenza or an infectious disease. Although the fourth-order replica regeneration is a form of second-order replica regeneration for a specific disease, simplified correlation data may be generated and handled to give priority to rapid response to a situation that requires urgency. Fifth-order replica regeneration may be generated as a response to a pandemic. Although fifth-order replica regeneration is a form of second-order replica regeneration for a specific disease, correlation data that is most suitable for the situation may be generated and handled with urgency from the viewpoint of the urgency and severity of the disease.

3. System Data Structure

3.1 Meta Model Description File

The meta model description file 14 may be provided to facilitate the use and availability of the diagnosis engine 20 in various application areas. The meta model is a model that describes a system to be subjected to diagnosis by the diagnosis engine 20 as a collection (set) of data or concepts that can be used by software. The system to be subjected to diagnosis by the diagnosis engine 20 may be a complex system composed of a large number of internal parts. Alternatively, the system may be a system in which internal parts have individual operating states that can be expressed as favorable, unsatisfactory, defective, or broken states, and where one of such states exceeding a certain boundary (limit) results in the system as a whole becoming abnormal or breaking down. Using the file, the states of internal parts (elements) and component parts are estimated from test data (observation data, measurement data, input data, and supplementary data) 11 of the target system, the test data 11 is re-estimated from variations of such internal states, and prediction and inference are performed from the differences (variance) between the actual test data 11 and the re-estimated test data. One target application of the support engine 20 is health care, and the system to be diagnosed is the human body.

3.2 Input Data

The input data for the diagnosis support engine 20 is the test data 11, which may be classified measurement data, such as blood, urine, exhaled breath, and an inner scan, and medical interview data. The measurement data may be categorized into data including items (parameters) that can be observed in general examinations and data including items (parameters) that can be obtained through more detailed examinations. The measurement data may include the heart rate when the body is under load, the time taken to return to a normal value, muscle mass, the degree of obesity, and information obtained by an inner scan. It is desirable for the information (medical interview data) obtained by a medical interview to be obtained by asking questions, such as a history of pancreatic cancer in the family, the subject's own medical history, and immunity, from a plurality of different points of view. The measurement environment of the measurement data may vary depending on the individual, and the medical interview data may play a role of compensating for differences between the measurement environments of individuals.

More specifically, the types of test data (examination data, inspection data) 11 for health care may include measured values for blood, urine, breath/skin gas, body temperature, visceral fat, subcutaneous fat, muscle mass, body fat percentage, estimated bone mass, blood pressure, blood flow, heart rate, autonomic nerve excitability and the like, image diagnosis results, information such as microRNA, ncRNA, DNA, and medical questionnaires. When the support engine 20 refers to past data as the test data 11, the data mentioned above will not necessarily all be available, but provided that some of the data is available, it will be possible to function in a manner where inference is possible.

The initial input format of the test data 11 may be composed of classifications, general names and abbreviations, numerical values, and units. The test data 11 may also include data such as an image diagnosis. Since the load when analyzing image data is large, the test data 11 may be produced by substituting descriptions of the internal parts of the body and the position, size, and progress of tumors/cancers. Regarding questionnaires, a questionnaire that is used by medical staff may be used as an initial medical interview. The test data 11 may include a result of additional tests requested from the verification engine 23, data of remeasurements made after two to three months, six months, or one year, the result of follow-up observations, and the like. The test data 11 may include results of repeated measurements performed several times in units such as two weeks and differential coefficient elements for judging whether the body is shifting toward a healthy state (that is, recovering) or is sick (deteriorating).

3.3 Output Data

The output data processing module 29 may output the output data 12 in a format that enables correlation with respective diseases to be found, focusing on internal states and substances that are markers relating to each disease and fundamentally from the viewpoint of a medical doctor participating in this project. The output data 12 may be summarized around an evaluation function and a correlation function for meta data. The accumulated amount of input data 11 and increases and decreases of each input item on a time axis may be evaluated individually or in groups, and the output data 12 may be outputted in a format with these trends added so as to enables correlation with the output data 12 to be found. For the system to be diagnosed, the output data 12 may be collected in a format that gives advice to a medical doctor, giving priority to a top 10 to 30 states that have been determined or estimated to be worthy of concern.

Regarding states that are estimated to not be significant regions of the target system, the output processing module 29 may also include potential worries and concerns for 1 to 3 years' time and 5 years' time in the output data 12 as messages. In the case of health care, since there are legal restrictions, there is a premise that checks will be performed by medical doctors. However, inference (prediction) of output data 12 is important in terms of diagnosis support, so that the output data may be provided with predictions made under certain conditions and may include probabilistic elements. For users who continuously use output of the diagnosis support engine 20 several times, a message that is more conscious of the difference with the previous output data 12 may be included in the output data 12.

The output data 12 may be configured to provide information on a plurality of properties. One object of this diagnosis support engine 20 may be to contribute to preventive care and preventative medicine by providing a tool that predicts and estimates health level and makes it easy to maintain a healthy state at the individual and/or company level. With conventional image diagnostic devices, and in particular health examinations performed once or twice, there are limitations on tracing, which means there are limitations when individuals manage their health. As the diagnosis support engine 20 and the system 10 that includes the diagnosis support engine 20 become widespread, it will be possible to deliver reminders for diseases (tumors, cancer, adult diseases, infectious diseases, and others) and a warning message when there is a risk of transition from a presymptomatic to a diseased state at an early stage. Doing so facilitates health measures and maintenance, which reduces the economic burden of individuals and makes it possible to reduce the medical costs borne by the nation as a whole. The diagnosis support engine 20 may be an engine with different specialties, and the output information 12 may be designed to be specific and detailed in proportion to an experience level. The output data 12 may be provided as information (health level: reassurances or warnings) together with probabilities of an active state, operating state, and defective state (disorder, fatigue, deterioration) of the internal parts of the body. In addition, advice information for future health promotion and countermeasures may be added for reference purposes in accordance with this information to the output data 12.

The data structure of the output data 12 generated by the output processing module 29 may be an audio output, text output, or image output. The output data 12 may be decided by linking the activation rate, operating rate (contribution, function) of the internal components of the system to be diagnosed, a matrix (numerical values) of multiple combinations thereof, and output indices and an auxiliary information table (addition).

The output data 12 may include information on health level. The health level may be simple, one-dimensional numerical information. If the health level can be classified into digestive organs, respiratory organs, throat, urinary organs, brain, heart, circulatory system, blood vessels, autonomic nerves, bones, spinal cord, and others, in view of changes over time, deterioration, improvement, sudden changes, and gradual changes, it is effective to use an expression format this is hierarchized as a tensor rather than vectors.

From the viewpoint of Western medical diagnosis and analysis, the output data 12 may include predictive estimation in which the interaction between internal components, changes over time in the interaction, and information on deterioration and improvement can be seen. Such information is also valuable in indicating the use level and usefulness of personal healthcare services. As one example, if the activity states of internal parts are quantified and changes over time are measured, this will enable users to more easily imagine the state from an image of physical acceleration and deceleration, and more clearly indicates recovery or deterioration of a diseased body. By giving individuals warnings and making plans having checked the effects of exercise and diet (dietary therapy), it becomes easier for individuals to understand.

A correlation equation that associates the input data 11 with the internal component elements (parts) may be written in the meta model description file 14. Also, information, such as the degree of health, the degree of presymptomatic disease, or the degree of disease that is finally observed as an external degree (stage, classification) may be written in the meta model description file 14. Typically, this is information that forms the basis for estimating a type and classification of disease. The output data 12 may include correlation between a plurality of input data (measurement values) 11, activity levels or disorder levels across internal components, and simple hints or guidelines for recovery. However, the output data 12 is typically information in the manner of advice from a medical doctor (MD), and may include a message that urges the subject to consult a hospital or a specialist.

The output processing module 29 may provide the output data 12 via a WEB server. The WEB server and the AI server may communicate using TCP/IP. If the library to be used is Python, a socket library may be used. During communication with the WEB server, text or files may be used. The method of giving display instructions to the WEB server side may be realized by storing the same table on the WEB server and the diagnosis support engine 20 sides and having the diagnosis support engine 20 side indicate index numbers. The content displayed by the WEB server may be image information, audio information, and/or text information. The diagnosis engine 20 may communicate with a plurality of other diagnosis support engines (including artificial intelligence) or with medical doctors (humans) via terminals in order to make highly accurate decisions. When doing so, communication may be performed using an AI protocol and an AI gateway. The AI gateway is necessary for communication with humans.

The diagnosis support engine 20 or the output processing module 29 may provide the output data 12 that includes information that is assumed to be of interest to the user, based on search and activity histories of the user indicating the user's greatest interests. The output processing module 29 may also include a function that allows a user to receive a communication key and use a service via the cloud by becoming a member of a specified rank in a specified healthcare platform. If the user is in possession of his/her own medical interview data, the user may receive an estimation result for his/her health condition from the diagnosis support engine 20 via a WEB server. The diagnosis support engine 20 may also provide health advice of a level that does not correspond to medical practice, together with the health condition.

The diagnosis support engine 20 or the output processing module 29 may include a function for connecting to a service via the cloud through an AI protocol and/or AI gateway in order to realize information exchanges between a plurality of engines and/or communication with a medical doctor. In order to maintain the greatest possible degree of symmetry, a plurality of diagnosis support engines 20 may be equipped with an AI uniform (an "AI shell" or an "AI suit") so that links with other AIs can be handled uniformly.

From application development, the output data 12 may be provided (published) by preparing a specific description as an API so that it can be called by a library. In this case, such exchanges can be performed by file or text-based communication. Connection to a WEB server may be performed for a plurality of purposes. These purposes may include performing communication for high-precision decisions according to an AI protocol. On the other hand, the purposes may include a step by step provision of information of concern to the user by estimating what information is necessary based on user searches and the like using a communication mechanism.

When a user becomes a member of a healthcare platform of a specific service company, the user may be allowed to enter a world of cloud-based services. An arrangement may be used where a user enters medical interview data via the cloud and is able to acquire a certain level of prediction from a diagnosis support engine 20 via the cloud. When there are limits on the WEB server, an engine that is capable of interactive dialog with the user via the cloud may be used on a platform handled by the diagnosis support engine 20. The diagnosis support engine 20 may be equipped with a landscape or navigation for reaching the entrance of a cloud service and a function for indicating the necessary information as guidelines.

Advice as to how to answer questions in a medical interview may be given in a questionnaire provided by a cloud service or the like. A system where a series of questions and answers from which information akin to a health map of the user or a mechanism that enables a plurality of engines to interact with answers to a medical interview may be implemented, and/or predictions are made via a plurality of diagnosis support engines 20 exchanging information with a medical doctor, including analysis of answers to the medical interview, via the cloud or the like, is effective.

### 3.3.1 Digital Avatar

The output data 12 may be a digital avatar provided by the avatar processing module 291. The digital avatar may be accessible on a typical cloud service, but it is also possible to use a configuration where the avatar is accessible via a restricted cloud service provided by a specified service provider, for example, a platform such as P-HARP where it is easy to maintain security. A plurality of digital avatars may be set for one person in the real world (RealWorld, RW). Also, the plurality of digital avatars may all have completely identical health care characteristics, and/or may have a plurality of possible variations of such characteristics. In virtual reality (VR), a plurality of VR spaces may exist and one avatar may be placed in one VR space before moving to a different VR cyberspace on the same dimension. When such movement is executed, the original VR space may be cut off, and/or a maximum number of moves may be defined. Only one digital avatar is subjected to control by the user and is "activated". All of the remaining avatars become shadows and may be automatically updated according to certain rules. In principle, access from the real world to virtual reality (cyberspace) is performed on a 1 to 1 or a 1 to N basis, but N to M may also be possible. As one example, this arrangement may be used as necessary on the system operator side due to security problems.

The VR spaces may be classified into categories such as: (1) DNA space (blood relatives); (2) Geographic space (country, city, region); (3) Working space (workplace, school, part-time job); (4) Family space (kin); (5) Exercise space (walking, running, sports), (6) Hobby space (music, reading, painting, art, yoga, meditation, video games); (7) Events (drinks with friends, competitions, dates, relocating, graduation, transfers, new careers, retirement, travel, vacations, marriage, childbirth, funeral, and others); (8) Physical characteristic space (age, gender, weight, height, chest circumference, waist, sitting height, visceral fat, muscle mass, others); and (9) Life/environment space (meals, work, study, exercise, sleep, hobbies, conversation, meditation). Expansion may be performed by taking settings from a limited space into another cyberspace. Even if a user's interests move to another space in virtual reality, basic values on the user (such as health, fatigue, stress level, health care parameters, and past data) may be automatically updated and inherited.

Digital avatars and virtual reality simulate how individuals who exist in the real world may change in various environments and situations, and can be viewed as a game where the user's own QoL (Quality of Life) can be greatly improved in the real world. Here, by performing various simulations from various angles, such as how the user's health level, fatigue level, stress, mental state and sense of fulfillment will be activated in the future and will move toward a healthier state or a deteriorated state, it is possible to provide a game that is one guideline on causes and how to improve the user's health in the future.

To calculate the level of matching and accuracy between the real world and virtual reality, it is possible for the user to periodically undertake measurement and medical interviews according to the advice of a supervisor and guidelines to improve the matching between the digital avatar and the user. Many sponsor companies may have a presence in virtual reality and be allowed to make proposals for improving the user's health. A similar system may be constructed in the real world, not only in virtual reality.

A key that is required for security (protection) when information is provided as a digital avatar may be issued by a digital avatar, a VR manager called a supervisor, or a "Key-Maker" dispatched for this role in the future. A Key-Maker issues a key for a time, place, and an ID of a digital avatar, which is then used to maintain security. However, the supervisor may periodically request a change of key. When one person (participant) in the real world is mapped as an avatar to a plurality of different virtual reality worlds, this is expected to result in improvements in comparisons with big data and estimation accuracy from various viewpoints. For a case where the user's close relatives (blood relatives) include one patient with pancreatic cancer, the risk of the user developing pancreatic cancer is higher than that of an ordinary person. As one example, in the family space (number 4 above) in cyberspace, estimation and advice based on such correlation information may be expressed in a more emphasized form. In the future, correlation and other hypotheses that require verification may be reflected in the inference engine 22 as one condition to be used. Since the AI engine 21 learns and develops by using the input data 11, which includes actual measurement data, and the replicas 13, predictions of pandemics or infectious diseases do not need to be given a stronger emphasis than necessary unless they reach a level where they become a social problem.

When the output data 12 is provided by the digital avatar in the cloud service, a security check is executed at a stage where the digital avatar is first entered. When diving from the real world into virtual reality, switching may be performed according to a technique called AR (Augmented Reality). This dive may be performed according to a method preferred by the user. A dive from the real world is a dive performed according to a method to which the user consents, and may be monitored by a security cyber agent. It is also possible to protect the system through security measures that use information that is only known by the user, the location (presence of the user), bio-authentication, or another means. Although it is possible to fake an appearance or disguise oneself in the real world to cause misidentification as the real user, in virtual reality or cyberspace where a plurality of digital avatars are active, in a state where the health care information belonging to a real person is inherited and observation from the outside is possible, it is possible to provide an environment where changes of observation forms can be easily made but tracking them is difficult. Access to the real world via a digital avatar can be performed in principle only by the real person, but if necessary, access may be performed by opening a hotline through a cyber agent. Since various items that exist in the virtual reality cyberspace also exist in the real world, to check the effect, a user may experience using an item in the real world and then bring the result into the virtual reality cyberspace via the user's own digital avatar.

3.4 Input/Output Correlation Table/Description File

The input/output correlation table/description file (correlation information database, correlation information) 15 is information used by a correlation processing module 27 and the inference engine 22. The table is generated based on reliable correlations that can be confirmed on the Web and information that has been checked by medical doctors (specialists and medical researchers) and is believed to be correct.

The input/output correlation table description file 15 classifies the types and numerical values of the input data 11 and creates a correlation table of internal states (such as the operating states and activation rates of each part), which as a result decides the output data rankings and accuracy (% probabilities) from the vast number of combinations. On the other hand, for cases where the internal states (variations) are known, the file is structured so that the input data 11 can be estimated. Here, a concept of prescriptions planning, based on the idea in current Western medicine that disorders, defects, damage, or incomplete functioning of internal body parts is connected to illness, may be reflected. At present, many medical devices and equipment in use have been developed to support this idea. Accordingly, the input/output correlation table description file 15 is preferably configured and styled to imagine such an environment and reflect it to the greatest degree possible. The correlation information 15 may describe a determination index indicating correlation for the current top 10 to 20 highest-ranked diseases, illnesses, infections, or the like.

4. Details of Each Module of Diagnosis Engine

4.1 Input Processing Module

The input processing module 25 prepares (arranges) the test data 11 so as to have a common data format for the inference engine 22 and the AI engine 21, and may also include a function of preprocessing the test data 11 according to predetermined rules. The input processing module 25 in the present embodiment includes an input data processing module 250, a preprocessing module 26, and a correlation processing module 27 that generates a map indicating the correlation between parameters included in the test data 11.

4.1.1 Input Data Processing Module

The input data processing module 250 may include a function for aligning the input data 11 so as to have a common data format for the inference engine 22 and the AI engine 21. By converting or organizing the input data 11 into a unified format to the greatest degree possible and performing preprocessing, blood data, urine data, inner scan data, and data on skin gas, breath, and medical interviews may be converted to a format that can be processed in a uniform manner. Also, via correlation processing, some out of the data may be marked (labeled), as one example to indicate at what level a numerical value of creatinine leads to a decrease in renal function or renal failure. In the case of diabetes, since the glucose level or HbAIC is often used as a reference, the progress of the disease can be estimated from these numerical levels.

When medical interview data is included in the input data 11, ambiguity will remain, and for this reason processing that converts from a plurality of questions into numerical values may be executed. The answers may be converted into numerical data as instructed in advance in the description file. In the case of breast cancer, it is necessary to configure the interview so that the breast shape, body line, and a certain volume (size) can be estimated. In particular, when estimating the activity level and functional contribution of internal components of a system, depending on the internal replicas 13b, there are cases where it is not possible to estimate from the measurement data included in the input data, and estimation may be performed entirely by preparing a plurality of interview data. In the case of pancreatic disease, if there is information such as the presence of a pancreatic cancer patient in the family or as a relative, the input data 11 may be generated so that the probability/risk rate of such disease is increased.

For the sake of processing, it is desirable for the support engine 20 to handle the input data 11 as numerical data to enable the data to be handled as uniformly as possible. The actual measurement data will be numerical values in the case of blood data, for example, but in the case of medical interview data, there will be ambiguity, making it necessary to convert into numerical data. As one example, if a questionnaire includes "Strongly applicable", "Applicable", "Not sure", "Not applicable", and "Not applicable at all", it is possible to convert "Strongly applicable" to 4, "Applicable" to 3, "Not sure" to 2, "Not applicable" to 1, and "Not applicable at all" to 0. However, in the case of medical interview data, it is desirable to use labels that indicate that the numerical values have been produced by converting answers to a medical questionnaire.

The input data processing module 250 may perform other processing, such as for preestablishing the correspondence between the input data 11 and components of the system (for example, internal parts of the body). As one example, the support engine 20 may be a support system that generates and uses a specialized AI engine 21 for each disease through learning. The input processing module 25 may include input processing for including, as the test data 11, the inference result of the specialized AI engine 21 and information resulting from exchanges performed by the engine 20 with an outside specialist, as part of a verification process for the inference result. By including additional information that is useful for inference from a plurality of different angles in the test data 11, it is possible to provide a support engine 20 that knows all about the system subjected to the diagnosis processing and that also has detailed knowledge in specialized fields.

The input data processing module 250 may generate a table expressing correlation when there is correlation between the values of the parameters included in the input data 11. The input data processing module 25 may generate a table representing the correspondence between each parameter included in the input data 11 and the component elements of the target system.

4.1.2 Preprocessing Module

The pre-processing module (conversion/statistics/other) 26 may include a function that converts the input data 11 that has different properties for the correlation processing module 27 to standardize the test data 11 and enable handling by the inference engine 22, the verification engine 23, the optimization engine 24, and the AI engine 21. As one example, although a substance called creatinine exists as one marker substance for impaired renal function, it is known that there are differences between numerical values of this substance and the degree of impairment in renal function depending on age and sex. This means that it does not make sense to simply compare numerical values unless conversion is performed and normalization processing is used. Without such pre-processing of measurement data from a medical point of view, it is difficult to perform simple comparisons. Regarding the blood glucose level, depending on whether the subject is fasting or how much time has passed since taking a meal, the situation for estimating the activity of insulin will be completely different. Normally, it is necessary to judge the data together with a medical interview and convert and normalize the data so that the meaning of the numerical values can be compared to a certain extent.

4.1.3 Correlation Processing Module

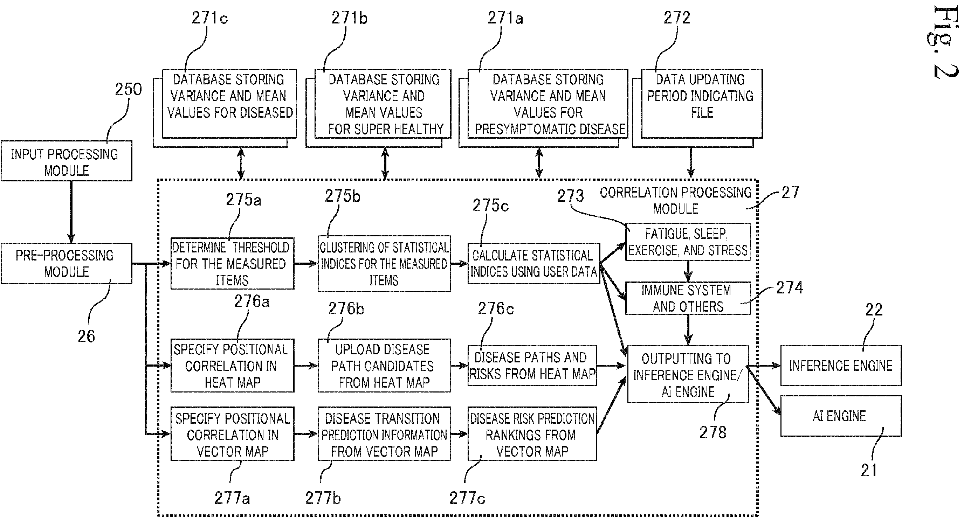

The correlation processing module 27 may actually perform a normalization process by converting the numerical values of the respective parameters included in the input data 11, and may map the data so that a meaning can be grasped from a comparison between the numerical values. FIG. 2 depicts an example of a correlation processing module that performs a correlation process on the input data 11 using a statistical method and provides the result to the inference engine 22 and the AI engine 21.

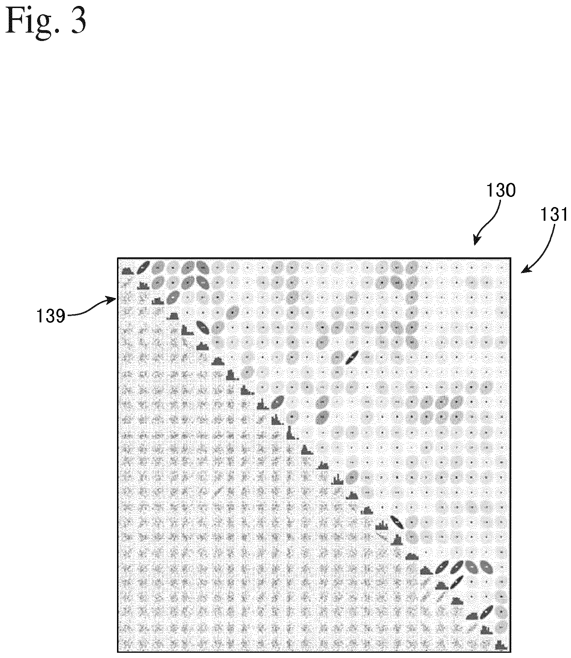

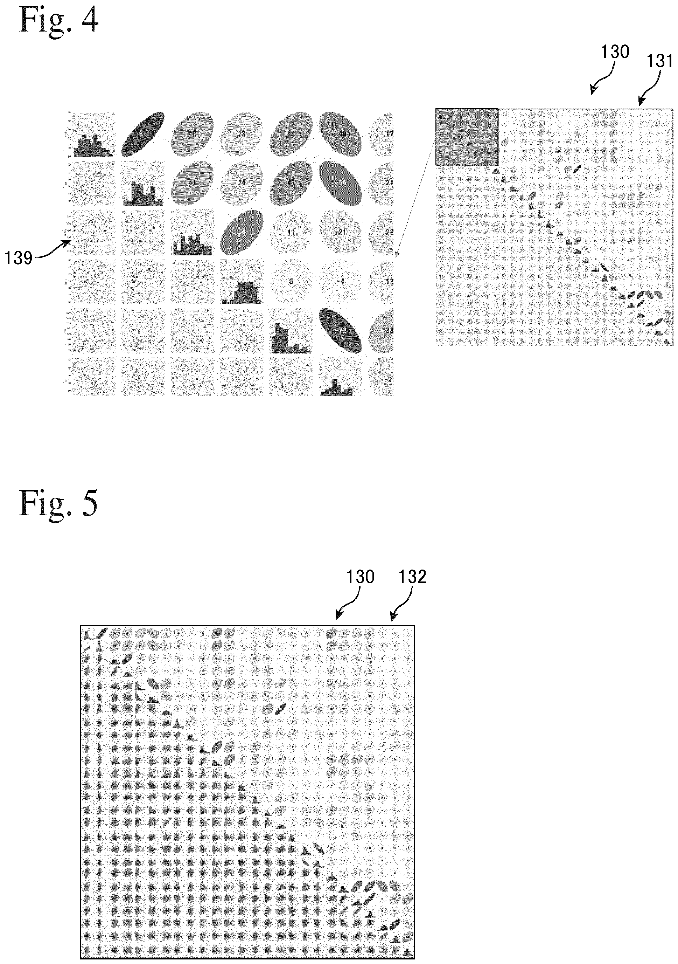

FIG. 3 depicts one example of a heat map 130 used in the correlation processing. FIG. 4 depicts an enlargement of a part of the heat map 130. The heat map 130 is an example of a heat map 131 for a normal state (a super healthy body). The heat map (a static matrix) 130 is a collection of correlation diagrams created from statistical data of test results that use, as a population, a large number of people (a large number target systems) such as people of each race, each gender, or each specific region in which a patient to be tested is included, or alternately the entire human race. One (one type of) heat map 130 represents one of several situations and states that humans included in that population may pass through, with a plurality of heat maps 130 being provided for a plurality of states. The heat map 131 for a normal state is an example of a result of analyzing the correlation between statistical data of test results of patients determined to be normal in a medical examination or the like, out of the population to which the patient belongs.

The heat map 130 is an aggregate (set, matrix) of a large number of scatter diagrams 139 that indicate the correlation between the respective test items and, in the heat map, test results (measurement points) of respective test items (parameters) of each of a plurality of patients who form the population to which the patient belongs, which are disposed on an X axis and a Y axis to indicate the correlations between test results of the test items. Note that an example of the correlation between items (parameters) in the test data (input data) 11 is correlation that uses scatter diagrams, that is, correlation in a scatter diagram produced by plotting numerical values of two different test items (parameters) out of the test data of a large number of patients as the X coordinate and the Y coordinate. The correlation may be correlation in which numerical values of a plurality of different test items are shown in multiple dimensions.

The content of a test includes measured values of blood tests, urine tests, breath/skin gas measurements, body temperature, visceral fat, subcutaneous fat, muscle mass, body fat percentage, estimated bone mass, blood pressure, blood flow, heart rate, autonomic nerve excitement, and the like, image diagnosis results, microRNA, ncRNA, and DNA, and also medical questionnaires included in a test. Such measured values obtained in tests are used in the scatter diagram 139. As examples, TG (neutral fat), HDL (HDL cholesterol), LDL (LDL cholesterol), FBS (glucose (fasting blood sugar)), HbAlc (hemoglobin A1c), AST (aspartate aminotransferase), ALT (alanine aminotransferase), gGT (γ-glutamyl transferase), ALP (alkaline phosphatase), LD (lactate dehydrogenase), TP (total protein), TC (total cholesterol), BUN (blood urea nitrogen), Cr (creatinine), eGFR (estimated glomerular filtration rate), UA (uric acid), RBC (red blood cell count), Hb (hemoglobin), Ht (Hematocrit), MCV (mean corpuscular volume), MCH (mean cell hemoglobin), and MCHC (mean corpuscular hemoglobin concentration) can be given as examples of measured items in a blood test.

The heat map 130 is one example of a static matrix. One example is a large number of scatter diagrams 139 for a large number of test items, including the test items listed above and other items, such as test items in a physical or cardiopulmonary examination like waist (abdominal circumference), BMI, BP-H (systolic blood pressure), and BP-L (diastolic blood pressure), for a patient judged to be in a super healthy state that have been laid out in two dimensions to facilitate image recognition. In the heat map 130, to show the correlation between the test items, a schematic in which all the numerical values are arranged as dots is provided in a lower left triangle and a schematic in which the correlation is expressed by graphs and numerical values is provided in the upper right triangle. When hardly any correlation is observed, + positive correlation indicated using small values, while negative values indicate negative correlation. Items where there is large correlation are expressed as large numbers.

FIG. 5 depicts a different example of the heat map 130. The heat map 130 depicted in FIG. 5 is an example of a heat map 132 in a quasi-normal state (presymptomatic, in Japanese "Mibyou"), and is an example of the result of analyzing the correlation of the statistical data of test results for a large number of patients, out the population to which the patient belongs, who were not judged to be normal or abnormal by a medical examination or the like. The expression "Presymptomatic" refers to people left in the measurement data/input data group after the super healthy people and the diseased people have been excluded. The expression "super healthy person" refers to a patient who exhibits normal values in all items (measurement data, input data, and medical interviews) in a clinical or medical checkup, and the expression "diseased" refers to a patient who has been determined to be ill by a doctor. This will normally be the state to which the largest number of patients can belong. It is possible to define "presymptomatic (mibyou)" as states in a plurality of stages from a state that is close to a healthy body to a state close to a diseased body, and it is possible to provide a heat map for each stage.

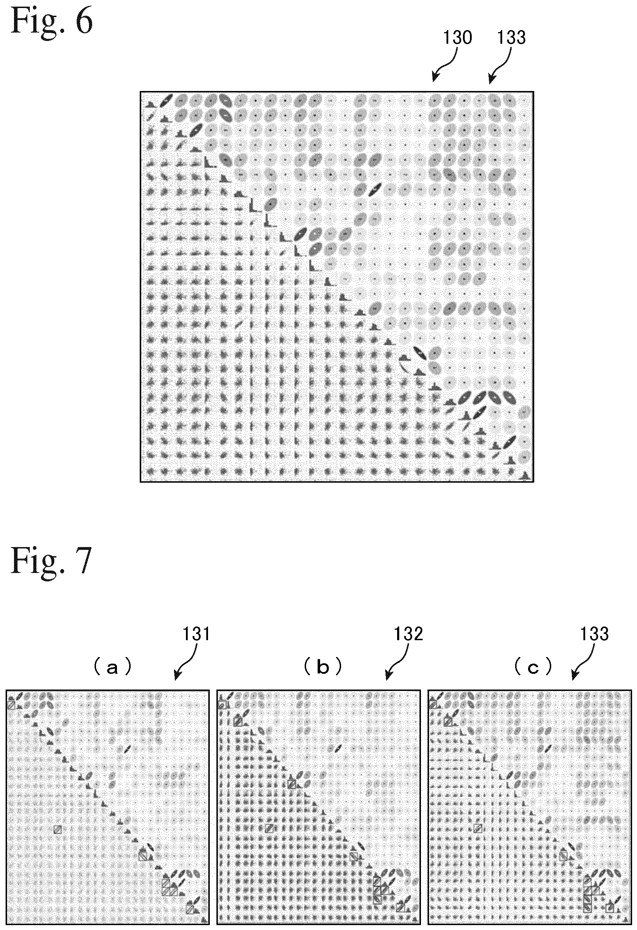

FIG. 6 depicts yet another example of the heat map 130. The heat map 130 shown in FIG. 6 is an example of the heat map 133 in an abnormal (diseased) state and is an example of the result of analyzing the correlation of statistical data on the test results of a large number of patients who have been determined by a doctor to have a specified disease, out of the population to which the patient belongs. The heat map 133 for diseased can be prepared for each stage of disease progression of a specific disease (condition).

In FIG. 7, the heat map 131 for super healthy, the heat map 132 for presymptomatic, and the heat map 133 for diseased have been laid out side by side. In reality, since there is a difference between the numbers of patients included in each state, the number of measurement values in the scatter diagrams included in the heat maps will vary, so that the heat maps that indicate each state are not complete. However, it can be understood that there are increases and decreases in the test items for which strong correlation is recognized in the respective states, and the heat maps (static matrix) 130 in which correlations between a large number of test items have been gathered differ in each state. Accordingly, it is possible to estimate the state during testing of each patient by comparing the relationship between a large number of test items included in the test data 11 of each patient with these heat maps 130.

Note that one example of the relationship between the test items is matrices of two-dimensional relationships, in which numerical values of two different test items, out of the numerical values of a large number of test items, have been plotted as the X coordinate and the Y coordinate in two dimensions and the matrices are laid out corresponding to a heat map. The numerical values of a large number of test items may be indicated by multidimensional positions (relationships) where each of a large number of test items are used as coordinates.

It is possible to prepare a heat map (static matrix) 130 for each state from super healthy to diseased.

Level 0: Super Healthy

"Super healthy" refers to a healthy body who at present has no known signs of disease risk. There are statistics that indicate that only a few percent of people who take medical checkups can be referred to as "super-healthy" or "super-normal".

Level 1: "Edge of Presymptomatic" or "Almost Healthy"

This level indicates subjects where compared to super healthy, the standard deviation value is in a range of 45 to 50, and one or more marker substances and/or combinations of input values indicating disease have been detected. When such subjects do not fall qualify as Level 2, they are handled as Level 1.

Level 2: "Transition to Presymptomatic" or "Unhealthy State"

This level is where the standard deviation value is in a range of 40 to 50, and two or more marker substances and/or combinations of input values indicating disease have been detected. This level is demarcated from Levels 1 and 3.

Level 3: "Typical Presymptomatic State" or "Actual Disease Risk"

This level is where the standard deviation value is in a range of 35 to 45 and three or more marker substances and/or combinations of input values indicating disease have been detected. This level is demarcated from Level 4.

Level 4: "Presymptomatic State Requiring Attention" or "Advise Disease Risk"

This level is where the standard deviation value is in a range of 30 to 40, and four or more marker substances and/or combinations of input values indicating disease have been detected. When possible in this state, it may be advisable to undergo a detailed examination at a medical institution. This level is demarcated from Level 5.

Level 5: "Clear Transition from Presymptomatic to Diseased State" or "Disease Risk Warning"

This level is where the standard deviation value is within the range of 20 to 35 and five or more marker substances and/or combinations of input values indicating disease have been detected. There is the possibility that one or more diseases have progressed to risk areas pointed out by a medical institution as a specified disease or complication. In this case, it is better to undergo a detailed examination without delay.

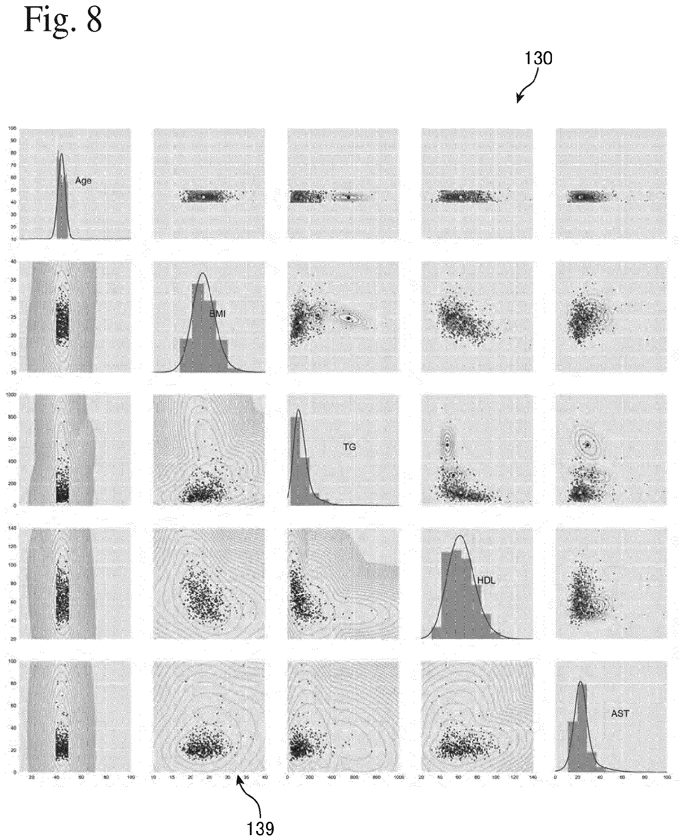

FIG. 8 depicts an example where the correlation of each scatter diagram 139 included in the heat map 130 is depicted with a probability distribution. In each scatter diagram 139, regions that have different statistical probabilities (standard deviations) are shown using contour lines. According to the inventor of the present application, cases where the correlation between the test data of the two test items gathered in the scatter diagram 139 can be expressed by one normal distribution and cases where the correlation needs to be expressed as a mixture of a plurality of normal distributions have been found. When the correlation is depicted as a mixture of a plurality of normal distributions, the normal distributions may be mixed on a one-to-one basis, or may be mixed at different ratios. By expressing the correlation between test items as a probability distribution, as described later, the heat map 130 can be used as a filter for illustrating the relationship between the test items of each patient. In addition, when the number of statistical data is small, a statistically significant number of samples may be virtually generated from the replicas to generate the heat map 130, or some or all of the scatter diagrams 139 of the heat map 130 may be supplemented by the replicas as well.

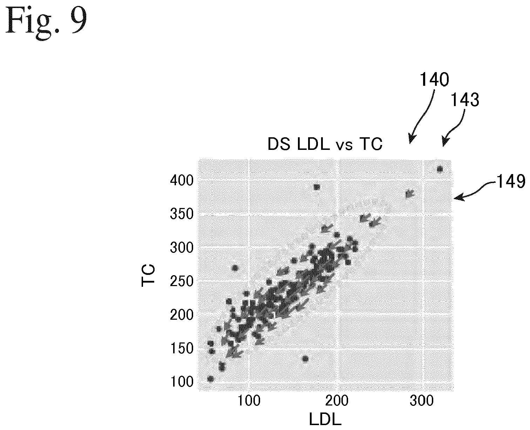

FIG. 9 depicts one example of a heat map 140 including vectors. This heat map with vectors (vector map) 140 is one example of a dynamic matrix, and depicts a vector-appended scatter diagram 149 that is one of diagrams included in the heat map 143 for diseased (non-normal). The scatter diagram 149 depicted in FIG. 9 shows the correlation between the total cholesterol level (TC) and the Low Specific Gravity low density lipoprotein (ND LDL), out of the test items. The vector map 140, which is one example of a dynamic matrix, includes a number of correlated displacements between heat maps (static matrices) 130 of each state, and reflects changes in the state over time in the population that includes the patient. The vector map 140 in this example shows displacements of the correlations in the scatter diagram 149 using arrows (vectors) indicating the directions and magnitudes.

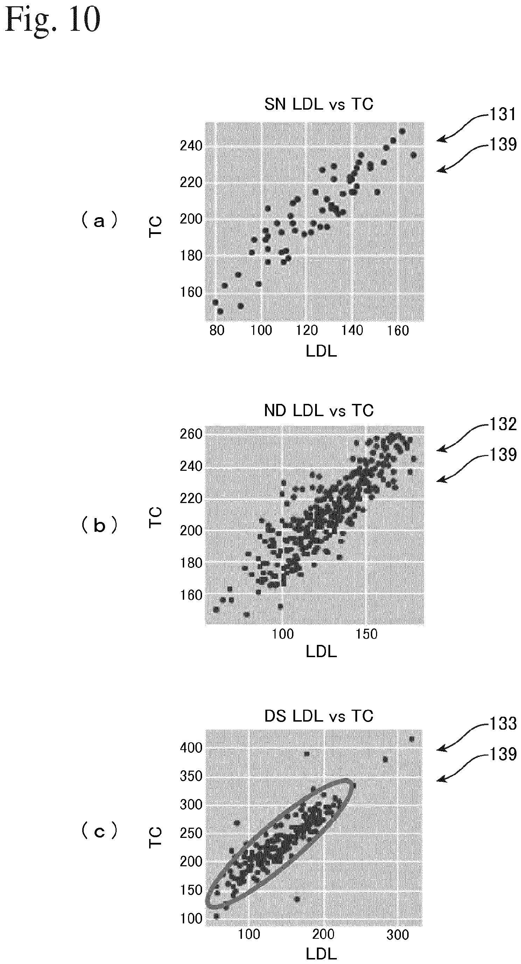

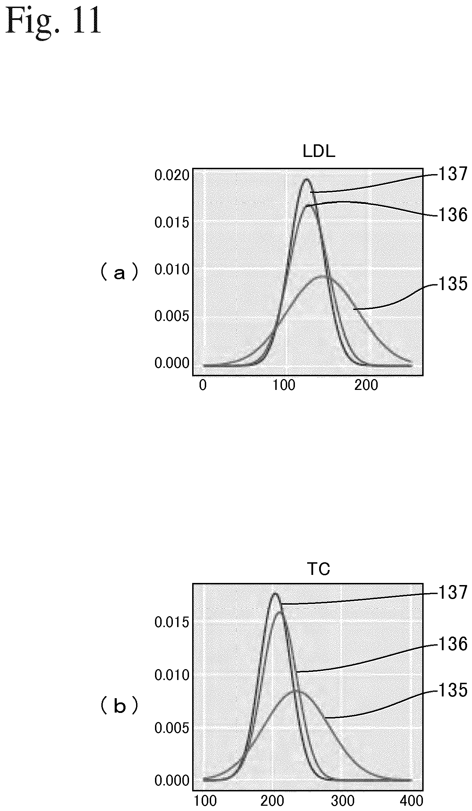

FIG. 10 depicts correlation (scatter diagrams) 139 between the total cholesterol (TC) and the low density lipoprotein (ND LDL) in the heat map 130 of each state. FIG. 10(a) depicts a scatter diagram 139 for super healthy, FIG. 10(b) depicts presymptomatic, and FIG. 10(c) depicts diseased. FIG. 11(a) depicts distributions of measured values of total cholesterol (TC) in each state, and FIG. 11(b) depicts distributions of measured values of low density lipoprotein (ND LDL) in each state. As depicted in FIGS. 11(a) and 11(b), the distribution 135 for super healthy is a curve with a low peak, the distribution 137 for diseased is a curve with a high peak, and the distribution 136 for presymptomatic is in between the two. Accordingly as depicted in FIGS. 10(a) to (c), the correlation between the total cholesterol level (TC) and the low density lipoprotein (ND LDL) plotted in the scatter diagrams moves from a state where there is almost no correlation between the scatter diagrams 139 of the heat map 131 for super healthy, via a state where correlation appears in the scatter diagrams 139 of the heat map 132 for presymptomatic to a state where high correlation is observed in the scatter diagrams 139 of the heat map 133 for diseased.

This means that it is possible to estimate the patient's condition by grasping, as the vector map 140, the correlation between a large number of test items at a second time of the next test result when time has passed from a first time point when the previous test result was obtained. Accordingly, by comparing a state estimated from the heat map 130 with a state estimated from the vector map 140, it is possible to make an estimation, including states that change dynamically. This means that by making an estimation using the heat map 130 and the vector map 140, the internal state of the system to be diagnosed can be estimated, including the changes over time, with higher accuracy and probability.

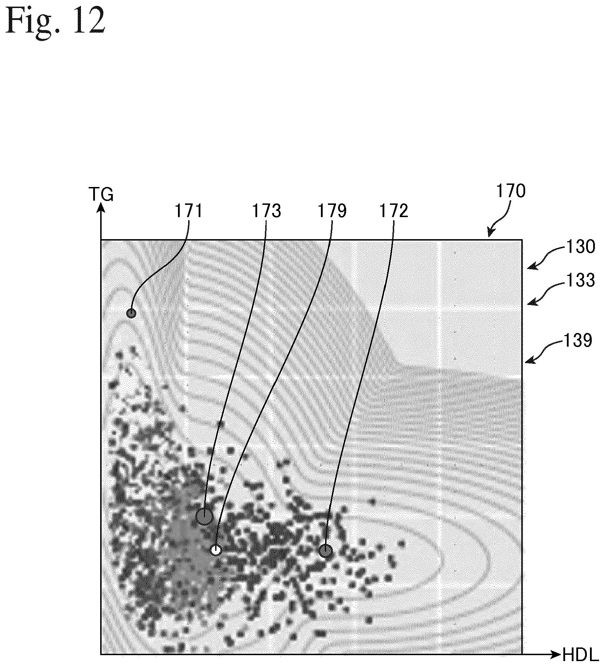

FIG. 12 depicts an example of an individual matrix 170 included in the heat map 130. This individual matrix 170 shows the relationship between triglyceride (TG) and HDL cholesterol (HDL) as the relationship between the test items included in an individual matrix 170. As one example, a relationship for three patients is plotted, and since the measurement value (relationship) of patient A is a position with low correlation (low probability), the value is displayed with a dot 171 with a small area. On the other hand, the measurement value of the patient C is displayed as a dot 173 with a large area because the value is at a position close to the correlation center 179 and therefore has high correlation (high probability). The measurement value of the patient B is displayed as a dot 172 with an intermediate area since the correlation is at a position with intermediate correlation (with intermediate probability). Accordingly, as one example, by using the heat map 130 of each state as a filter, the first test results of the patient A are converted into a plurality of individual matrices 170 including images composed of a large number of dots that have the same position but different areas. If the patient is different or the test result is different for the same patient, the positions of a large number of dots will differ and the area of each dot will also differ. Accordingly, for each test of each patient, an individual matrix 170 with different images in each state will be generated. By performing image recognition processing on images included in the individual matrices 170, the learning module 210 of the AI engine 21 is capable of learning to estimate a state corresponding to the test results of a patient. As the learning progresses, the AI engine 21 becomes capable of comparing with similar images of multiple past patients and estimating the present state of the patient.

As depicted in FIG. 12, the measurement data of each individual is on several different contours (showing locations that coincide with positions of people with similar tendencies) with respect to the heat map (presymptomatic level 3). The size and color (brightness) of the dots may be filtered and displayed in proportion to the Euclidean distance from the most common center value (where the ratio between the mean values of two axes and the standard deviation is 50:50). If a dot is close to the representative center, the AI engine 21 recognizes it as a large-sized dot, but if the dot is far from the center, the AI engine 21 recognizes it as a small-sized dot. Since the XY axes (that is, two axes, two dimension) are used, an expression of two dots of respectively different sizes (areas) is eventually recognized as a pattern indicating the feature. However, as described above the representation of dots may use color or brightness. As another representation, it would be conceivable to handle the data as a vector quantity given as (sizeTG, sizeHDL), for example. In this case also, by changing the size of dots in accordance with the Euclidean distance from the center, it is possible to perform enhancement processing to clearly show this position in the correlation and to facilitate the judgment by the AI engine 21. For the vector map 140, enhancement may be performed by reducing the vector magnitude when a vector is positioned at a location in the vector map with low density (that is, a rare case) and increasing the vector magnitude when a vector is positioned at a location with high density. The AI engine 21 may perform learning with a vector map 140 in which the positions of a large number of vectors or dots are different and the vectors have difference sizes, as images, and estimate dynamic transitions in the states.

Returning to FIG. 2, in the module (block) 275a, the correlation processing module 27 determines a threshold value for the measured items included in the inputted test data 11. More specifically, it is determined for each individual user whether each item in the test data 11 exceeds a reference value and a cut-off value specified in the external definition files 271a to 271c. In a database 271b that stores variance and mean values for super healthy, statistical indices for data relating to super healthy are stored using data on users and subjects that have been collected so far and data in an external database. Data on super healthy is important when conducting detailed analysis of a "healthy" or "presymptomatic" (level 1) to predict and estimate disease risk. This is because it is possible to make more accurate and higher-precision predictions compared to when data on diseased or data of a presymptomatic group is used.

In a database 271a that stores variance and mean values for presymptomatic ("mibyou"), the statistical indices for data related to the presymptomatic group are stored using data on users and subjects that have been collected so far and the data in an external database. The concept of presymptomatic ("mibyou") has a wide distribution from presymptomatic level 1 to presymptomatic level 5 and depending on the purpose, is provided so that prediction and estimation of disease risk and the health levels can proceed with the assumption of changes between groups. In a database 271c that stores variance and mean values for diseased, the statistical indices for data related to diseased are stored using data on users and subjects that have been collected so far and the data in an external database. A data updating period (deadline) indicating file 272 for the external definition file may also be included.

In addition, in a module 275b, clustering of statistical indices of the measured items is performed. Clustering of super healthy, presymptomatic, and diseased is performed for individual users based on the determination result of the thresholds of each input data. For super healthy, there is one or fewer items that deviate from the reference value. Presymptomatic ("Mibyou") is between super healthy and diseased. There are presymptomatic level 1 to presymptomatic level 5, and diseased, which is defined as having five or more items that exceed a cutoff or abnormal value. Next, in a module 275c, statistical indices are calculated using user data. For each data (blood data, urine data, vital data, autonomic nerve level, medical interviews, and other test data) of a plurality of users stored in an external database, a statistical index is calculated for each clustering target, by specifying, using an external definition file, whether the probability model of a normal distribution or a logarithmic distribution is to be used for each item, and the statistical indices are stored in the database 271b which stores the variance and mean values of super healthy, the database 271a which stores the variance and mean values of presymptomatic, and the database 271c which stores the variance and mean values of diseased.

In addition, in the module 273, a determination is made regarding fatigue, sleep, exercise, and stress. It is known that fatigue, sleep, exercise, and stress have are extremely high correlation with immune system activity at presymptomatic levels 1 to 5. For this reason, statistical metrics are calculated from data on the subject or user, the activity level of the immune system, future disease risk and its potential paths, and the potential for recovery toward health are further calculated from the fatigue, sleep, exercise, and stress items.

Also, in the module 274, the immune system and other factors and life pattern are determined. Separately to estimating the immune system levels from fatigue, sleep, exercise, and stress, by analyzing lifestyle patterns, disease risk, the time and/or period of shifts or transitions to a diseased state, or the potential for recovery toward health are predicted and estimated.

In parallel with the above processes, the module 276a specifies positional correlation in the heat map. By using the data of the presymptomatic group, the current position of the data of the user or subject is identified. Also, as correlation processing, predictive estimation is performed for the direction in which the disease path is likely to shift or move in the future. However, there is no time-based analysis of how long the period of the disease risk is. Such analysis is executed using the vector map. Next, in the module 276b, disease path candidates are uploaded from a heat map. Using the data of the presymptomatic group, the current position of the data of the user or subject is specified, and candidates for potential future disease risks are identified. In addition, in the module 276c, disease paths and risks are determined from the heat map. Out of the disease path candidates that have been listed up, analysis and predictive estimation are performed by limiting the degree of risk to a number of diseases.

In addition, in parallel with the above process, positional correlation in the vector map is specified in the module 277a. By using the data of the presymptomatic group, the current position of the user or subject data is specified. Also, as correlation processing, predictive estimation is performed for directions in which a disease path has a risk of shifting or moving in the future. After this, in the module 277b, disease transition prediction information is generated from the vector map. In the vector map, information on the immune system level may be partly included, so that it is possible to estimate future disease transition predictions from the user or subject data. In addition, in the module 277c, disease risk prediction rankings are generated from the vector map. From the heat map information and the vector map, shifts or transitions to a disease risk are predicted, and if there is past user or subject data, risk rankings of disease rinks are estimated along with the disease transition time of the previous stage.

After these processes, the module 278 performs output processing directed to the inference engine and/or the AI engine. As the output sent to the inference engine 22, it is possible to prepare the position of the user or subject being diagnosed, with the numerical information on disease risk and health recovery which has been specified based on statistical processing and/or the immune system level (activity level). The information may be prepared so as to also estimate, through generation and comparison of heat maps and vector maps, the time taken to transition or shift as a disease risk. The output sent for the AI engine 21 may be centered on heat maps and vector maps, with it being possible to incorporate immune system level information in the vector maps.

4.2 AI Engine

Artificial intelligence, or "AI", is now commonly applied to application areas that were difficult for conventional procedural programming languages, and has increasingly attracted attention due to the results it has achieved. The application areas where there are presently high expectations for AI are the five areas of language processing, image processing, audio processing, control technology, and optimization and inference, where high value-added implementations have been announced. At present, AI has already reached a certain level, but is expected to achieve more advanced performance in the future. Any type of artificial intelligence that is currently available or developed in the future can be applied to the AI engine 21. Several main types of AI engines currently being developed and their characteristics are described below.

In an SVM (Support-Vector Machine), classification is performed at locations where the margins between teaching data are maximized. SVM has a high generalization ability, and it is possible to design features through dimension reduction that uses principal component analysis. The decision tree is classified using a feature with the largest amount of mean value information when the teaching data is divided. The causal relationship between inputs and outputs is easy to understand, and features may be designed by dimension reduction using principal component analysis. K nearest-neighbor classifier performs classification according to a majority out of K teaching data labels located near the input data. Although it is easy to adapt new teaching data to the classifier, the amount of calculation required to classify input data tends to increase every time the teaching data increases. A hierarchical neural network learns and classifies features and the like from teaching data using back propagation. Although features are automatically learned from teacher data using back propagation, there is a tendency that as distance to the input layer falls, the gradient disappears and learning becomes difficult. In deep learning, features and the like are learned and classified from teaching data by a self-decoder. Although the features are automatically learned from the teaching data using the self-decoder, no particular explanation of the causal relationship between inputs and outputs appears.

Reinforcement learning (for example, Q-Learning) learns optimal behavior by giving a reward to the behavior of an agent in a certain state. The behavior that the agent learns can be changed according to how the reward is given, which means that it is possible to learn the optimal behavior by designing the rewards. In unsupervised learning (for example, K-means), clustering is performed on data whose structure is unknown. In state with no teaching data, clustering is possible based on the degree of similarity between data, and reasons for clustering data groups may be thought up by a human if necessary.

One form of AI suitable for the AI engine 21 of the diagnosis support engine 20 is deep learning. Methods that use deep learning have produced very useful results in the field of image recognition. It is desirable to apply this ability to the diagnosis support engine 20, which makes it possible to provide a highly precise AI engine 21. The AI engine 21 proceeds with analysis of health level by performing continuous measurement, as one example, in units of 2-3 weeks. The accuracy of predictive inference becomes clear through continuous measurement and health analysis for 2-3 months, 6 months, or 1 year. There are three key points to improving diagnostic accuracy: improving the self-prediction accuracy of the optimization engine 24 (that is, investigating the correlation of the difference data from the correlation given as an initial condition in the meta model description file and heuristically raising the accuracy of predictive inference); performing one to several additional tests (further precise measurements and medical interviews): and execution every two to three weeks and tracing the changes over time to perform verification. The performance index may be designed with an emphasis on throughput and turnaround time with respect to the amount of input data. In particular, for big data analysis, sufficient performance to complete the processing of one input data within a few minutes may be provided as a system configuration that can ultimately guarantee processing of one hundred thousand to one million transactions within a few hours to a few days. The accuracy of the prediction and estimation of the AI engine 21 can be reduced to a level where the error rate is less than 10% and there is no serious oversight, compared with the case where a doctor judged. In addition, it is possible to realize predictive inference that can achieve about 95% reliability.

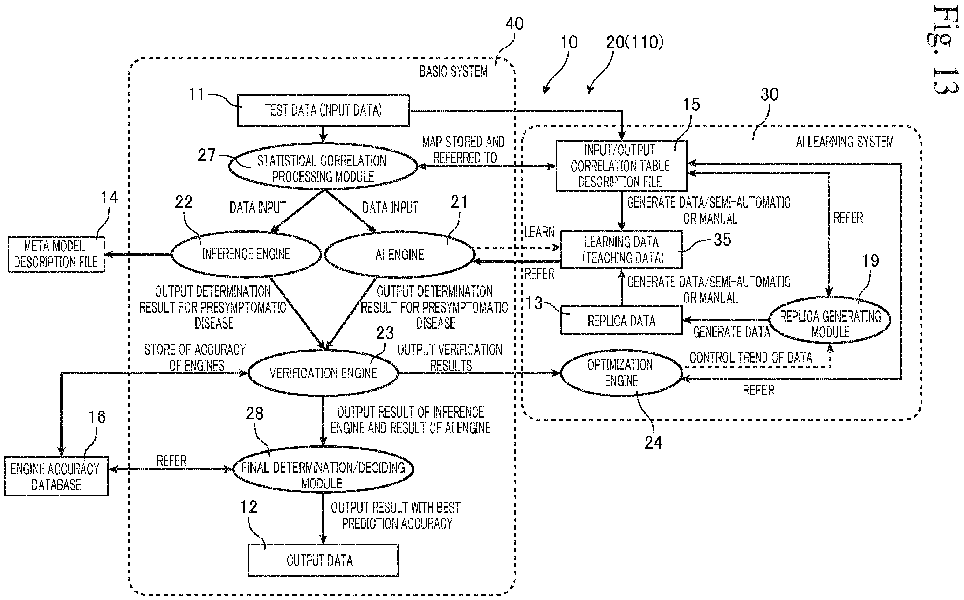

FIG. 13 depicts a learning image of the AI engine 21 in the support engine 20. The support engine 20 may include a basic system 40, in which the inference engine 22 and the AI engine 21 estimate the internal state of the target system to be diagnosed based on the test data 11, and a learning system 30 of the AI engine 21. The learning system 30 includes a path that sets the test data (input data) 11, which is the user data (real data), as the teaching data (learning data) 35 and a path that sets replicas 13 generated by the replica generating module (replica generator) 19 as the learning data 35. In accordance with an instruction from the optimization engine 24, the replica generator 19 generates replicas 13 of an amount and a range that cover where the test data 11, which is real data, is insufficient, thereby promoting learning by the AI engine 21. That is, the control method of the support engine 20 includes providing a replica optimization policy for the replica generating module 19, based on the accuracy of the state estimated by the AI engine 21, which has been verified by the verification module 23.

The verification engine 23 verifies the accuracy (correctness) of the estimation result of the inference engine 22 and the accuracy (correctness) of the estimation result of the AI engine 21 and stores the results in the engine accuracy database 16. The final determination/deciding module 28 outputs the estimation result with the higher prediction accuracy as the output data 12. The final decision determination module 28 may be equipped with a module or logic that outputs the estimation result of the inference engine 22 with priority while a state where the accuracy of the estimation result of the AI engine 21 is lower than the accuracy of the estimation result of the inference engine 22 continues and outputs the estimation result of the AI engine 21 with priority while a state where the accuracy of the estimation result of the AI engine 21 is higher than the accuracy of the estimation result of the inference engine 22 continues.

4.3 Inference Engine

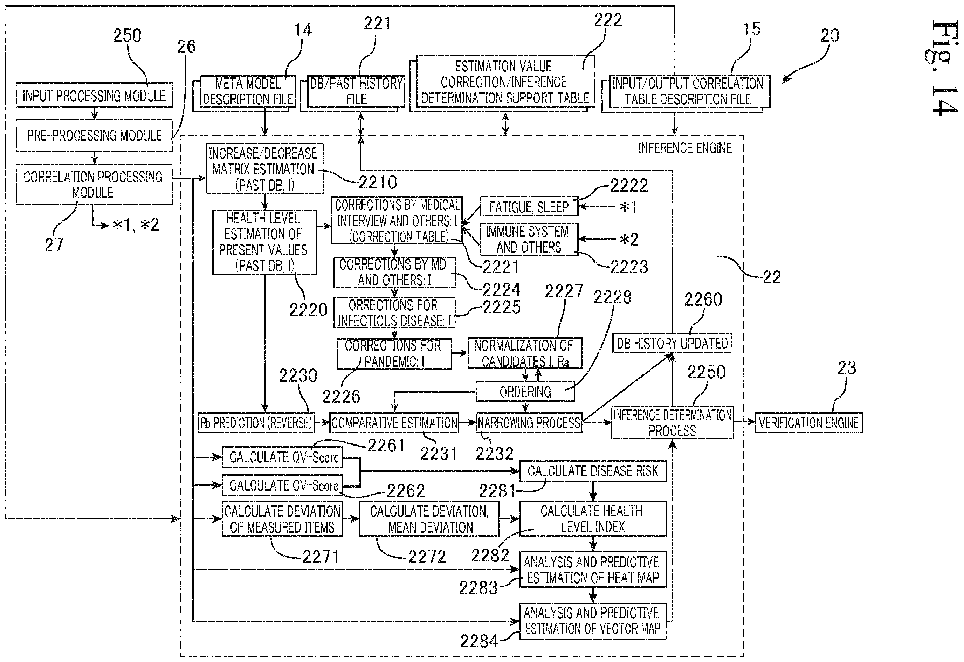

FIG. 14 depicts a more detailed configuration of the inference engine 22. As the meta model information that configures the inference engine 22, the description file 14 may include one or more evaluation function expressions used to execute inference and information for narrowing variations of the generated replicas. Rules for preprocessing the inputted test data 11 may also be set in the input/output correlation table description file 15. The inference engine 22 may generate replicas, and/or may infer the internal states from input data and/or medical interviews.

Replicas are also generated by the replica generating module 19. Although the replica generating module 19 has a function of executing replica generation independently, it is possible to change the variation of replica generation according to instructions by the optimization engine 24 of the diagnosis support engine 20. The replica generating module 19 is mainly executed based on statistical data and a replica generation ratio designation table, and may be constructed with no prediction function. The main function of the replica generating module 19 is to generate input replicas, internal replicas, and reproduction input replicas that have the designated contents. This replica generation may differ to the inference engine 22 in that such replica generation of these replicas from the input data (measurement data, medical interviews, and the like) is performed in certain ranges of replicas that can be imagined within the ranges that can be obtained from such information.

The inference engine 22 executes inference from the generated replicas, and/or input data or medical interviews. The inference engine 22 may narrow down the estimated states while referring to the correlation table 15, a past history 221, tensor amounts, and advice information from a medical doctor to create inference candidates for a plurality of possibilities. Medical interviews may be performed by incorporating methods, such as question and answer, to check whether there is a genetic problem without DNA data being used. As one example, if someone has a person with pancreatic disease in his/her family or as a relative, the rules of the inference engine 22 may be set so that the possibility of pancreatic disease is inferred or predicted higher than if no such person existed. Also, if someone who has responded to a medical interview in the past is suspected of having a weak immune system function, the rules of the inference engine 22 may be set so that inference and prediction are performed for the immune system activity level taking into account recovery periods from experiences of high fever and/or diseases.

The rules (prediction model) implemented in the inference engine 22 may be defined by evaluation functions that compare variations of internal replicas with a plurality of regenerated input replicas. The inference rules of the inference engine 22 may be fed back to the optimization engine 24, including the results of the verification engine 23. The learning model by which the AI engine 21 learns may include a method of performing optimization based on this feedback. One of the rules implemented in the inference engine 22 may be that the differences between the input data (actual measurement values and the input replicas) 11 and a plurality of regenerated input replicas regenerated from the internal replicas are compared with distributions (numerical value: o) and the candidate with the smallest differences is set as a correct candidate as an internal replica indicating the internal state of the target system. A plurality of internal states indicated by a plurality of internal replicas may be quantified and a plurality of candidates may be provided as probabilities. These internal states may include "super healthy", "presymptomatic", "diseased", "tumor", "cancer", "sick", and the like.

That is, the inference engine 22 may generate a plurality of candidates for an internal replica as a hypothesis for the internal state from the input data 11 and statistical data.

The inference engine 22 may regenerate the input data 11 from a plurality of candidates generated by the replica generator 19 and detailed data, such as correlation between data, regional characteristics, age, and medical history. For the plurality of internal replicas 13b that have been generated by the replica generator 19, the inference engine 22 may use detailed data to estimate which internal replicas 13b are closer to the actual situation. The inference engine 22 may also pass inference results for the activity rates, defects, and failures of internal components to the verification engine 23.

The main operations of the inference engine 22 may include the following three parts.

The first part is inferring the states of organs from the input data 11 based on an external table.

The second part is inferring the input data (regenerated replicas) once again from the inferred states of the organs (internal replicas).

The third part is determining what input data appears to be highly accurate out of the inferred input data (regenerated replicas) based on the actual input data and the replica data, and re-evaluating the internal replicas.

For the part where the states of organs are inferred from the input data 11, the inference engine 22 may perform inference with reference to the external tables 14 and 15 according to the format described below. As the rules when making inferences, three cases, that is, where indices and the states of the organ (activity level, defect, abnormality) have one-to-one correspondence (that is, a one-to-one model), one-to-N correspondence (a one-to-N model), and an M-to-N model (including complications, complicating disease) may be considered.

In the case of the one-to-one model, where there is only one corresponding internal element (or internal component: an organ or something else), when the presence of one marker substance that can specify an abnormality has been confirmed, the state of the organ can be estimated by knowing only one index. This means that a decision can be made by searching indices associated with organs for a corresponding (appropriate) label in order from the top.

When there is one or more marker substances that are estimated to be abnormal and one or more internal elements (internal components: organs or something else) that correspond to such marker substance(s), that is, the one-to-N model or the M-to N-model, it is possible to refer to weightings of each index described in the external correlation table 15 and estimate the states of the organs individually with consideration to a plurality of indices. Estimates where judgement is difficult, such as in the case of complications, may be performed later. Also, when there are a plurality of candidates, it is possible to use an implementation where each marker substance is assumed to be linear and changes with respect to an index according to a coefficient. That is, in simple terms, when there are a plurality of failure factors, a method may be used where multiplication by coefficients is performed according to the symptoms and the results are added. If the immune system or other recovery factors are involved, the influence of the coefficients may be independently reduced for each factor. As a result, it is possible to use an implementation where simultaneous equations are solved for the time being by raising and lowering factors positive coefficients and negative coefficients independently of the factors related to such coefficients.

Next, regarding the part where input data is inferred again from the state(s) of organ(s), for the case of the one-to-one model, an external table is used to determine possible values of each index from the state of the organ.

Finally, in the part that compares the inferred input data with the real data, the distance between the inferred input data and the real data is compared, generation of input replicas is executed again with the top five inferred input data that have the shortest distance to the real data set as the inference result, and the top five input data and the regenerated input replicas are passed to the verification engine 23 as the output.

An example configuration of the inference engine 22 will now be described in more detail. In the module 2210 depicted in FIG. 14, estimation with increase/decrease in matrix is performed. When considering the health level, a rapid increase (or rapid decrease) is considered to be a sign that there is a risk of sudden change. The present examination data may be amended and corrected with consideration to increases and decreases in view of changes over time, for past measurement data of an individual. Increases and decreases in the examination data are defined as increase/decrease in matrix, that is, a matrix that is appended with a date and has increases and decreases based on past measurement values for an individual as data. Since the inference engine 22 wishes to discover where sudden deterioration has occurred, it is necessary to use coefficients so as to give consideration to changes in data of the most recent two to three weeks rather than past data from two to three years ago. Ultimately, it becomes possible to make estimations by looking at only increases and decreases in the matrix.

In module 2220, estimation of the health level is performed for the current values. Here, rule-based estimation is performed based on the correlation data. Since the correlation data 15 can include one-to-one or one-to-many estimation, and many-to-many estimation, it is necessary to change the estimation method for each correlation data.

Since it is believed that various factors have an influence on the examination data, it is necessary to make corrections in order to perform inference correctly. This correction is performed using the correction table 222. The correction table 222 may be created together with a doctor or specialist. Based on the content of the correction table 222, values in the examination data may have difference values added or subtracted, or correction coefficients may be applied. The correction method may be a method where the examination data is corrected by selecting an index indicating an internal component. When an internal index is selected, content indicated by an index may be corrected according to a description table.

In more detail, in the module 2221, medical interviews and other corrections may be made by self-reporting by the subject. This includes self-reporting (2222) of fatigue, sleep, and the like, and self-reporting (2223) of the immune system and other factors. In the module 2224, it is possible to make corrections by a medical doctor and others and to make corrections according to feedback from the attending physician, doctor or other medical personnel. In the module 2225, correction of infectious diseases may be performed according to a warning or the like from the Internet or a medical institution. In the module 2226, a pandemic may be corrected according to a warning or the like from the Internet or a medical institution.

In module 2227, normalization of candidates is performed. All test values in the test data/input data 11 may be normalized as a standard deviation. The normalization may be changed according to male or female and age. The standard deviation may vary depending on the enterprise, region, city, region, country, and environmental conditions, and the handling used in the normalization may also be changed depending on the purpose.

In the module 2228, the data or information are ordered or ranked. During ordering, a ranking is assigned to each organ or part of the body in order to know in detail which organs have a disease risk worthy of attention and which parts are related to maintaining health. As a method of assigning the rankings, a method of arranging organs or parts with a low level of health (that is, a high disease risk) in descending order may be used.

In the module 2230, reversely estimated data may be generated for the data before correction. The replica generator 19 reversely estimates the input data/test data 11 with respect to the activity levels of the internal organs and parts of the body, and calculates possibilities within a certain range. Since one test data/input data 11 does not necessarily correspond on a one-to-one basis to one organ or part, and there are many cases where the test data 11 includes a numerical value linked to a plurality of locations, the reversely estimated data may be gradually corrected (in stages). The value obtained by reverse estimation of the input data/test data 11 is R ("Reverse" or "Ra" or "Rb", where Rb is the reversely estimated data before correction, and "Ra" is the reversely estimated data after correction). In reverse estimation, reference is made to the health level (immune system level, stress, fatigue, sleep, exercise, and the like) and correlation data 15 for the health level of each organ and part that have been estimated from the examination data 11 and the correlation data 15, to perform reverse estimation for the input data/test data 11.

In the module 2231, comparative estimation is performed. A comparison is made between the reversely estimated data before correction and the reversely estimated data after correction. This comparative estimation is performed for each individual organ or part of the body. The role of the comparative estimation is to check in which range the difference between the Ra and Rb predicted values calculated by the replica generator when correction is performed and the tolerance are included. The degree of correction is also adjusted based on this result to improve accuracy.

In the module 2232, a narrowing process is performed. Narrowing down of candidates with high disease risk is performed for organs and parts (elements) in the body. The criteria for narrowing down may be organs and parts for which there is little discrepancy, for example, in a positive +3 level, and an increasing trend of numbers when past differences are viewed, and organs and parts with the numerical value being low, where the numerical value of normal people is 0 or more, and the disease risk having increased according to differences with the past.

In the module 2250, an inference determination process is performed. The role of the inference determination process is to perform an output process for data that has been narrowed. In more detail, "I" (the internal state, internal replica, or internal organ and part) and "R" (reverse estimation, regeneration replica) generated by the inference engine 22 are passed (outputted) to the verification engine. Information on why this estimation was determined, that is, information on the basis of the estimation may be outputted to other processes. As one example, when there are ten organs with a 90% degree of abnormality, but only five out of them can be outputted, it is determined which organs or parts are to be outputted as part of the inference determination process. That is, when narrowing could not be performed in the narrowing process, the inference determination process may be performed. In more detail, it is possible to refer to statistical processing, past data, and, in order to investigate the relationship with heredity, the disease history of family members or relatives. Data that is passed to the verification engine 23 may be directly passed inside an application without going through an external file.

In the module 2260, updating of the history database (DB) 221 is performed. The present input data/test data of an individual may be registered as a standard deviation value in the history database 221 to enable future reference.

In addition, the inference engine 22 performs calculation of the QV-Score (scoring of a categorical variable) in the module 2261. At the inference engine 22, it is possible to calculate the disease risk and the health level of each organ. The QV-Score estimates disease risk from biomarkers (binary). In particular, disease risk may be estimated from medical interview data entered by the user. The calculation may be executed separately for each out of various cancer risks (pancreatic cancer, liver cancer, lung cancer, colon cancer, and other cancers).

In the module 2262, the CV-Score (scoring of a continuous variable) is calculated. The CV-Score is used to calculate disease risk, and in particular, disease risk is estimated from a user's biomarkers (continuous variables). The calculation may be executed separately for each out of various cancer risks (pancreatic cancer, liver cancer, lung cancer, colon cancer, and other cancers). In the module 2281, the disease risk may be calculated through conversion of the CV score and the QV score based on a predetermined distribution ratio.

In the modules 2271 and 2272, a mean deviation and a deviation value are calculated. Based on a disease relationship matrix (a table showing the association between the data and diseases (organs)), deviation values of items related to the evaluation target are squared and weighted, and the mean value of the sum is calculated. As necessary, weightings may be applied according to a definition file to the individual deviation values.

In the module 2282, the health level index is calculated. The following formula may be used to convert from the mean deviation to a health level index (Health Score). The formula is given below.

$$\text{Health Score} = 100 - \text{SDmean} \times 25$$

SDmean: Mean deviation (mean value that has been weighted based on deviation in items related to the evaluation target)
Health Score: If the calculated value is 0 or below, the score is rounded to 0.

In addition, in the module 2283, analysis of the heat map and predictive estimation are performed. As described above, a heat map is obtained by calculating the XY correlations (found by normalizing the biaxial correlation) for all of the input data (blood data, urine data, vital data, autonomic nerve level, medical interviews, and other test data) and laying out in the two dimensions. In addition, all XY correlations may be converted into image information reflecting differences in color depth (density, intensity) so that the disease risk (red) and health (blue) become clear. Since the XY correlations are used, an image in which the test data are reflected as shades (depth variations) of red and blue on the X side and the Y side is obtained, thereby producing an image on an individual level (personalized image) depicting one kind of disease risk/health state. As one example, when a value is close to the mean value, a white-colored image is obtained. In reality, an image in which red, blue, and white are mixed is created corresponding to the individual's health level and disease risk.

When such images are accumulated and classified, a huge transition map for disease risk and health state is created. For disease risk, the presymptomatic level 5 has the highest risk of disease being actualized, and with a prominent trend toward a specific disease occurring in reality. The presymptomatic level 4 is the state before a shift to the presymptomatic level 5. The presymptomatic level 3 is on the way to health or disease. The presymptomatic level 1 is close to a healthy body and the presymptomatic level 2 is midway on a transition to the presymptomatic level 3. When there is a risk of disease, even at presymptomatic level 1, the trend appears as shades (contrasts, depths) are dark and light red. The predictive estimation of disease risk may specify a static position (which does not consider change or movement) from using the position of the health level/disease risk, and from this position, it is possible to predictively estimate the directions in which the disease risk can shift or transition in the future.

In module 2284, vector map analysis and predicative estimation are performed. As explained earlier, the vector map is a map in which past input data/test data (deviation values) are regarded as changes over time and the amounts of change are expressed as vectors toward disease risk, while considering the immune system level based on the autonomic nervous level, fatigue, stress, sleep, exercise, and the like (it is also possible to decide the degree of immune activity by directly testing leukocytes/NK cells, B cells, T cells, and/or macrophages). It is possible to search for a vector that matches the subject's input data/test data (deviation value) 11 and estimate what kind of danger of heading toward a disease risk exists from the current state for the user. Alternatively, it is possible to predictively estimate whether the subject is recovering and returning to a healthy state.

4.4 Verification Engine

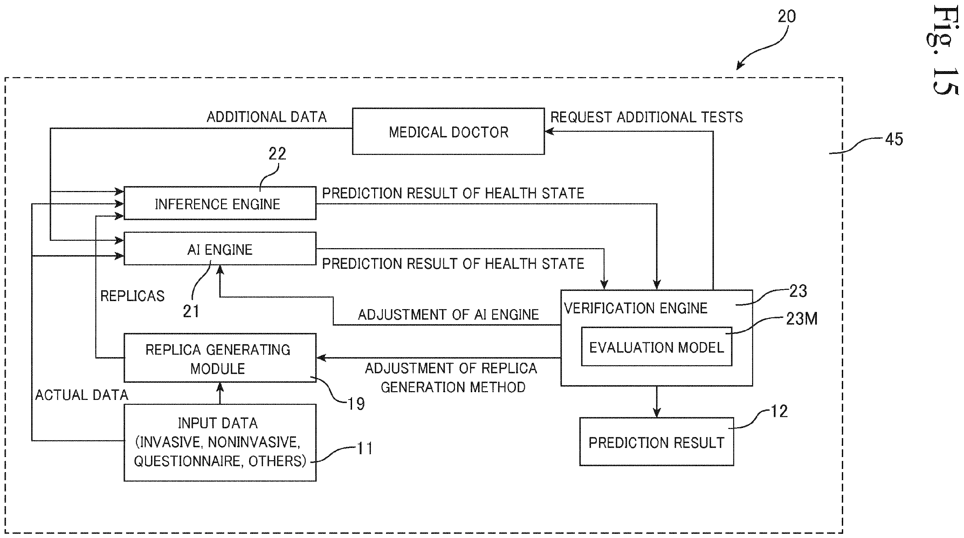

FIG. 15 depicts an overview of the verification cycle 45 executed by the verification engine 23. The estimation accuracy (correctness) of the inference engine 22 and the AI engine 21 may be improved according to data of an additional test requested by the verification engine 23 or the health status of a patient or a presymptomatic person after the passage of time. The additional test data conforms to testing specified by the medical doctor. For example, a test item where disease may be present is executed. However, when a subject is considered healthy, all of the results will be favorable even when testing is executed multiple times.

As the evaluation model 23M used by the verification engine 23 to evaluate the estimation accuracy of the inference engine 22 and the AI engine 21, it is possible to find the difference between the test data 11, such as measurement data, a medical questionnaire, and statistical data, and input replicas 13c that have been regenerated from the internal replicas 13b, and select the data with the most favorable distribution. The evaluation model 23M operates even when the AI engine 21 is operating as the main unit. When there are a plurality of prediction and inference candidates and the difference between them is small, additional measurement is executed and the result is re-evaluated by taking the evaluation model 23M into consideration. The verification engine 23 may have a function that is operable when there was a similar case in the past on a past time axis, to reevaluate such pending case that could not be decided in the past, and make a decision. The verification engine 23 may also have a function that is operable when there is a problem for an evaluation (for example, clarification is not possible), to perform an additional medical interview for items where there is doubt (unclear). The verification engine 23 may have a function of making changes to the input replica generation method and the output method of the AI engine based on the verified data.

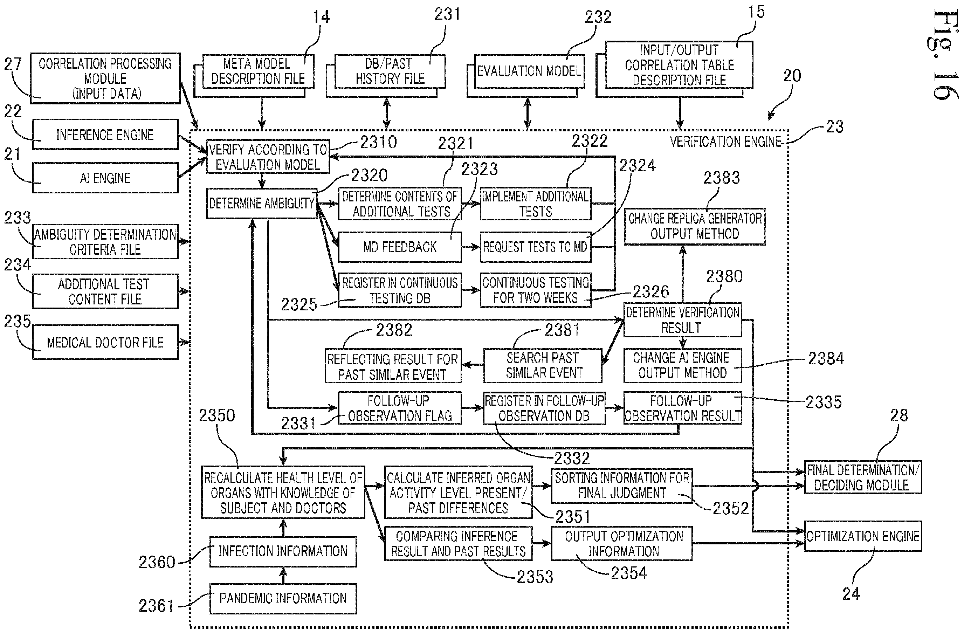

FIG. 16 depicts an example of a more detailed configuration of the verification engine 23. In the module 2310, the verification engine 23 performs verification of the output of the inference engine 22 and the AI engine 21 (that is, values that estimate states) according to the evaluation model 23M. By loading the evaluation model 23M described above or an evaluation model 232 that is externally defined, verification of the input data 11 and the inference result (estimation result) of the inference engine 22 or the AI engine 21 is performed. In the module 2320, the ambiguity of the estimation result is determined. The ambiguity determination module 2320 that has received the verification result attempts to improve the accuracy (correctness) of the inference results of the inference engine 22 and the AI engine 21 based on the verification result. In the ambiguity determination module 2320, it is possible to determine the points (items) where ambiguity remains from the result of the verification module using the evaluation model. The determination criteria may refer to an ambiguity determination criteria file 233 that is an external definition file.

If it is determined that the accuracy is low (that is, the ambiguity is high), the following three additional verifications may be performed. The additional verifications are (1) additional tests that asks the user to answer more detailed questions, (2) a test request that asks a medical doctor to make a diagnosis, and (3) continued testing where testing is performed continuously for two weeks. The ambiguity determination module 2320 may perform the additional verifications in order from number (1), and the verification results may be passed (outputted) to the verification result determination module 2380 when the ambiguity disappears. The ambiguity determination module 2320 may pass (output) the verification result determination module 2380 a record of what kind of ambiguity determinations were performed for what verifications.

The module 2321 determines the content of the additional tests. The additional test content determination module 2321 determines what additional test content is to be performed based on the determination of the ambiguity determination module 2320. The content of the additional tests may be decided with reference to the additional test content file 234, which is an external definition file. The module 2322 is a module that controls the execution of the additional tests, and transmits the content of the additional tests to the cloud service (P-HARP) side. At the cloud service, additional tests are performed based on the content received from the follow-up test execution module (verification engine) 2322.

The module 2323 selects a medical doctor (MD). The selection module 2323 requests the medical doctor to perform the additional tests based on the result of the ambiguity determination module 2320. The request of MD may be determined by referring to the MD file 235, which is an external definition file. The module 2324 controls the request for an investigation to the MD. The inspection request module 2324 makes an actual examination request to an MD defined in the MD file 235.

The module 2325 is a registration module 2325 for registration in the continuous testing database 2326. If ambiguity still remains even after an MD has been requested to perform an examination, the user may be registered in the continuous testing database 2326 so that the inspection is continued for another two weeks. The user registered in the continuous inspection database 2326 inputs the results of the testing that has been continued for two weeks. If the ambiguity disappears during the continuous testing, the testing may be terminated at that point, and the user may be deleted from the continuous inspection database 2326.

The module 2331 is a module that generates a follow-up observation flag. If the ambiguity remains even after testing has continued for two weeks (continuous testing), the follow-up observation flag module 2331 adds a follow-up observation flag. By regularly observing the follow-up, it is checked whether the ambiguity has disappeared. The module 2332 registers the user, for whom the follow-up observation flag has been set by the follow-up observation flag module 2331, in the follow-up observation database. The follow-up observation result module 2335 confirms whether the ambiguity has disappeared as a result of regularly observing the follow-up. If the ambiguity has disappeared, the observation of the follow-up may stop at that time, and the user may be deleted from the follow-up observation database. The follow-up observation database and the follow-up test database may be included in the past history file database 231.

The verification result determination module 2380 receives the result when the ambiguity determination module 2320 has determined that the ambiguity has disappeared. The verification result based on the evaluation model, the input data and verification content received by the ambiguity determination module 2320, and a record of the ambiguity determinations performed so far are received, and a verification result based on the evaluation model is passed onto the final decision determination module 28.

The verification result determination module 2380 may pass (hand over, output) a record of verification contents and ambiguity determination to a past similar event search module 2381. The past similar event search module 2381 checks whether there is a user (event) in a similar state based on the verification contents and the record of ambiguity determinations from the follow-up observation database and the continuous testing database. In the follow-up observation database and the continuous testing database, it is desirable for verification contents and a record of ambiguity determinations to be kept for each user. When a user who is in a similar state is present in the follow-up observation database and the continuous testing database, a record of a similar user and a similar verification content and ambiguity determination are passed to the past similar event result reflecting module 2382. Based on the received record of a similar user and a similar verification content and ambiguity determination, the past similar event result reflecting module 2382 executes the ambiguity determination so as to reflect the ambiguity determination of the similar user results in the evaluation model in a previous evaluation model. If the ambiguity disappears at this point, the continuous testing and follow-up observation may stop.

The replica generator output method changing module 2383 may change the output method of the replica generating module 19 based on the verification result and an externally defined file. The AI engine output method changing module 2384 may change the output method of the AI engine 21 based on the verification result and an externally defined file.

In the verification engine 23, the estimation result selected by the verification result determination module 2380 and the reasons for determination may be provided to the final determination/deciding module 28 and the optimization engine 24, and an estimation result or the like may also be provided after determining the activity level and disease risk of organs. The module 2350 recalculates the health level of organs and the like, based on the information and knowledge of the subject and doctors. In order to perform the verification, the inference engine 22 and the AI engine 21 may calculate the activity level and disease risk of the organs to be predicted. In the module 2350, the activity level and disease risk of organs calculated by a doctor may be treated as the activity level and disease risk of organs to be predicted by the inference engine 22 and the AI engine 21. At this module 2350, a doctor may perform calculations regarding the activity level and disease risk of organs based on data inputted from the user, infection information 2360, and pandemic information 2361.

The verification engine 23 may output information that enables the final determination/deciding module 28 to determine which prediction out of the inference engine 22 or the AI engine 21 is to be used. An inferred organ activity level present/past difference calculation module 2351 calculates, for the data inputted by the user, the differences between the respective prediction results of the inference engine 22 and the AI engine 21 and the result of the subject/doctor's knowledge organ activity level recalculation module 2350 and store the result in the database 231.

The result of the inferred organ activity level present/past difference calculation module may also be stored in the database 231 including the past results. The final judgment information sorting module 2352 calculates information (such as the mean value of the present result and the past result) required by the final determination/deciding module 28 based on the past results and the present results of the inferred organ activity level present/past difference calculation module.

The verification engine 23 may collect information that is used for optimization when such information can be collected. The past inference result comparison module 2353 compares the current inference result with the past results and collects information (transitions in inference accuracy and the like). The optimization information output module 2354 provides the optimization engine 24 with information that is to be used in optimization, from the information collected by the past inference result comparison module.

In this way, it is desirable for the verification engine 23 to include a module 2320 that determines whether there is ambiguity in the state estimated by the inference engine 22 or the AI engine 21, and modules 2321 to 2325 that acquire additional information from outside according to the ambiguity. It is desirable for the additional information to include at least one out of feedback by a diagnosis expert on the target system to be diagnosed, a follow-up observation, and results of additional tests.

4.5 Optimization Engine

The role of the optimization engine 24 is to improve the inference accuracy (correctness) of the inference engine 22 and the AI engine 21. For this reason, the optimization engine 24 optimizes the replica generator 19 based on the verification result of the verification engine 23. In this optimization, it is possible to consider what to do when differentiation cannot be performed and how to avoid getting stuck at a local solution. Examples of optimization methods include a GA (genetic algorithm), simulated annealing, and the like. A mathematical method may be used, and approximation may be performed using coefficients, such as Lagrange's undetermined multiplier method or a Fourier series. The actual optimization method is specified by the meta description file 14.

The information for optimization may include a static normal distribution and may include a Bayesian estimation. Errors may be reduced by performing Bayesian inference, based on the time direction and additional tests, and then making corrections. When some of the predicted variations are changed and a predicted value is determined in the future, feedback may be provided. Correction may be performed on the assumption on the real future being used and the present being used as the future.

4.6 Final Determination/Deciding Module

The final determination/deciding module 28 makes a final determination based on the result verified by the inference engine 22 and/or the verification engine 23. Final determination/deciding criteria may be generated in advance as a metafile, and the final determination/deciding module 28 may make a final determination/decision based on decision-making criteria and the verification result. If it is determined that the verification result favorably reflects the real data according to the decision-making criteria, the final determination/deciding module 28 accepts the verification result. On the other hand, when it is determined according to the decision criteria that the verification result does not reflect the real data, or when it is determined that there are a plurality of hypotheses as the verification result, the final determination/deciding module 28 has verification performed again at the verification engine 23.

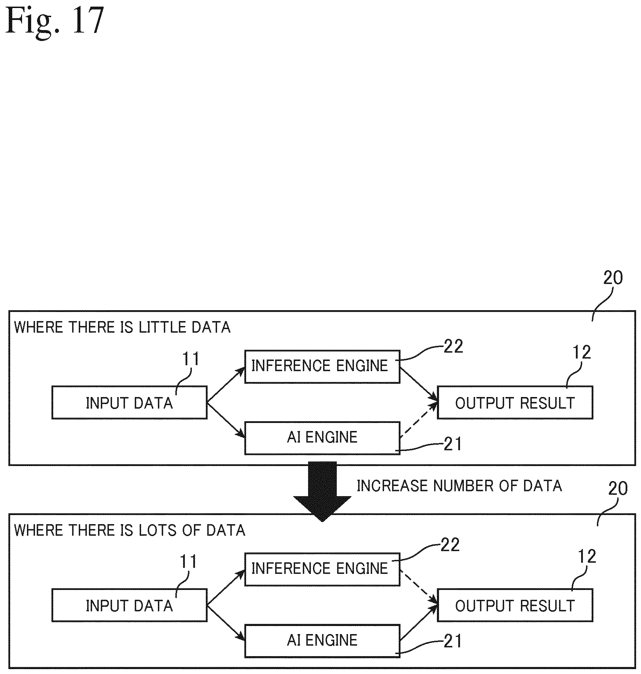

As depicted in FIG. 17, the final determination/deciding module 28 may include a function that decides which inference is to be prioritized based on the inference accuracy of the AI engine 21 and the inference engine 22. Accordingly, a control method of the support engine 20 that includes the AI engine 21 and the inference engine 22 includes a stage ST1 that outputs the state estimated by the inference engine 22 with priority over the state estimated by the AI engine 21 and a stage ST2 that outputs the state estimated by the AI engine 21 with priority over the state estimated by the inference engine 22, according to the verification result of the verification engine 23.

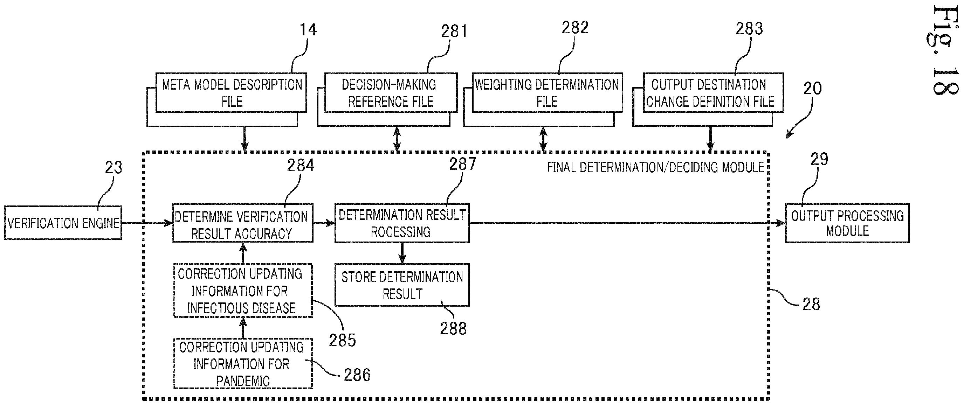

FIG. 18 depicts an example configuration of the final determination/deciding module 28. A verification result accuracy determination module 284 refers to an external definition file 281 for the determination method for accuracy, and calculates the accuracy of the verification result using the actual verification result. The determination result processing module 287 compares the accuracy of the respective verification results of the AI engine 21 and the inference engine 22. For the method to be used for comparing, such as comparing weighted values, it is possible to refer to the decision-making reference file 281 and a weighting determination file 282 that are external definition files. From the determination result, the decision whether to output to the output processing module 29 or to have the verification engine 23 perform verification again may be made with reference to the output destination change definition file 283. The determination result storage module 288 stores the determination result in an external database.

When infectious disease information 285 has been provided from the Internet, a medical institution, the government, or the media, such information may be received and used as one reference material for the final judgment in the predictive estimation of disease risk. Likewise, when pandemic information 286 has been provided from the Internet, a medical institution, the government, or the media, such information may be received and used as one reference material for the final judgment in the predictive estimation of disease risk. Such information may be received and reflected in the judgment, especially when a disease spreading over a wide range can be foreseen.

4.7 Output Data Processing Module

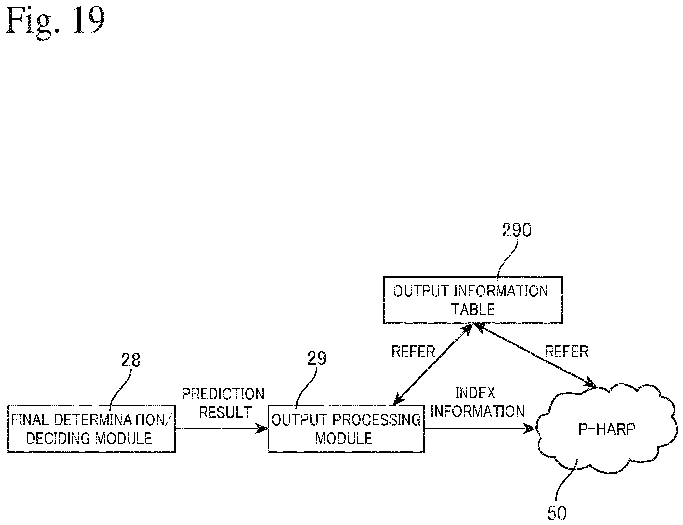

FIG. 19 depicts the processing outline of the output data processing module 29. The output data processing module 29 may be equipped with a function which, when presenting a prediction result to a user, modifies the method of expression and the like to avoid confusion with actual medical practice and then transmits to a WEB server. The types of output data include audio information, image information, and text information. In accordance with instructions from the inference engine 22 or the AI engine 21 and the final decision module 28, the output data processing module 29 may request index information for an appropriate output information table 290 from a cloud service (P-HARP/Web server) 50. Due to the nature of a meta model, this index information may be realized by a method according to the rules on the output side of a control table.

4.8 Replica Generating Module (Replica Generator)

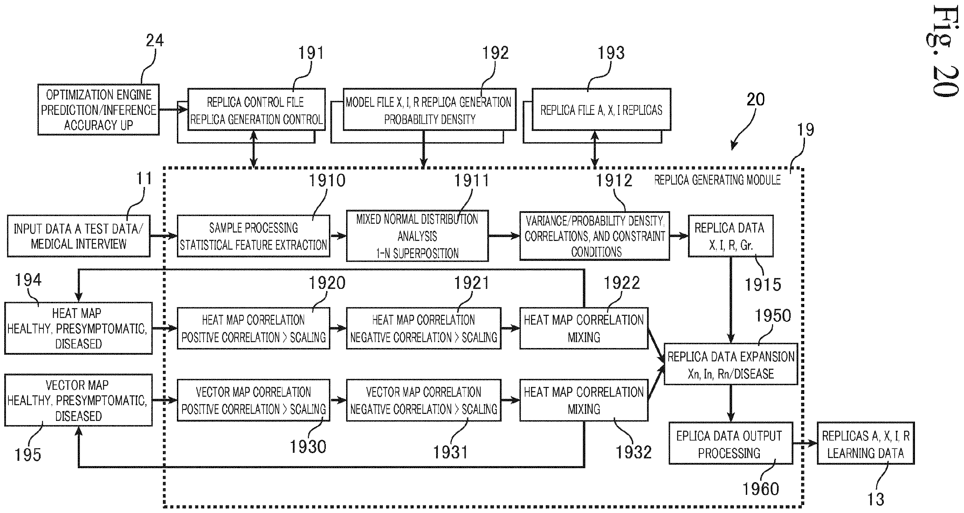

FIG. 20 depicts an example configuration of the replica generating module 19. A module 1910 performs sample processing (sampling) and extraction of statistical features. At the beginning, the module 1910 starts a process of confirming the input data or test data for which replicas are to be generated by sample processing and feature extraction. The processing of the module 1910 may be preprocessing, and directs replica generation together with an instruction from a replica control file 191 about what kind of replica generation is to be executed. By using the replica control file 191, the optimization engine 24 may realize replica generation control that improves the prediction accuracy. If clear instructions are not given in the replica control file 191 and the model file 192, extraction of statistical features may be performed through the module 1910, and preparation for the execution of replica generation may be performed within the range of constraint conditions from the replica generation probability densities of the XY correlation information.

A module 1911 performs mixed normal distribution analysis and 1-N superposition. It is statistically known that as the quantity of input data/test data 11 increases enormously, this increase will lead, according to the "law of large numbers" and the central limit theorem, toward to a normal distribution. However, in cases such as where the number of test data 11 is not sufficient, where the samples are limited to certain groups, or where XY correlations of statistical features are found for all of the input data/test data 11, a normal distribution may not appear clearly. In particular, to find XY correlations, it is possible to introduce an analysis method that analyzes statistical features with variance and/or probability density as a two-dimensional distribution in which a plurality of normal distributions are superimposed. By doing so, it is possible to adopt an analytical method with two-dimensional features that is synthesized using random numbers based on the constraint conditions in which positive correlations, negative correlations, and/or a plurality of normal distributions that have different magnitudes are combined to execute the analysis keeping with the features. There are cases where this can be simple analysis with a single normal distribution, but depending on the XY correlation data, there are also cases where the form in which a plurality of normal distributions for different magnitudes of 2 to N overlap may be a feature of the statistical data. The analysis mentioned here may be executed by the module 1911.

The module 1912 performs processing that finds variance/probability density and features such as correlations and constraint conditions. Features of statistical data include probability density and variance (mean, representative values, and the like), and by investigating the XY correlations of all input data/test data 11, it is possible to clearly find positive correlations and negative correlations. The obtained features and constraint conditions may function as constraint conditions when executing the generation of replicas. It is possible to assume a simple rule, for example, only a normal distribution, and execute replica generation for probability density in accordance with this rule. However, there is a high probability that replicas that deviate from the real data will be generated. For example, when the human body is considered as one system, if a problem occurs at one organ or part, it is recognized that in the input data/test data 11 relating to that part, the numerical values may get better or worse in the form of related groups. This means that the individual pieces of data (the values of the parameters included in the test data 11) are not necessarily independent but are often interrelated. In some cases, the relationship may be expressed by converting increases and decreases in the values of a plurality of parameters into a matrix or matrices. In this case, it is desirable to perform replica generation in a form where the statistical features are maintained. The module 1912 may perform a preparation process for achieving this.

The module 1915 generates replica data (X, I, R/Gr). Module 1915 generates, based on the input data/test data 11, pseudo input data/replicated data (X: measurement data, input replicas), the activity level of internal organs and parts corresponding to the input information (I: internal replicas), and also pseudo-replicated data (R: reproduction replicas, regenerated replicas) to be regenerated by looking at such internal I values. The generated replicas may be stored in the replica file 193.

In this support engine 20, the learning system 30 includes a function that provides a path that makes it possible to perform corrections by comparing a difference between real data and data obtained through a series of predictive estimations with certain assumptions and from information that is currently known. Artificial intelligence (the learning module 210 and the AI module 21) performs predictive estimation of potential rules from an enormous amount of data and contributes to improvements in accuracy. The learning system 30 functions as a path through which predictive estimation can be verified, and enables analysis of a complex system to proceed autonomously or through self-learning. In the current medical system, it is not possible to analyze the relationship between body parts (elements) or organs and the input data/test data 11 in a simple way. The learning system 30 that uses replicas makes it possible to add a structure that is very similar to a framework that observes a complex system as components of the system and estimates the relationship with diseases. It is therefore possible to easily track internal states indicating how the complex system is dealing with a disease or diseases. When a disease risk starts to appear, the module 1915 may contribute to further increases in the accuracy of predictive estimation of disease risk by generating replicas by linking the parameter fluctuations that are constrained by that group (Gr.) level (disease group).

A module 1920 performs scaling processing (for disease) for heat map correlation (heat map) 194, and in particular for positive correlation. The heat map 194 depicts the XY correlation for all parameters in the input data/test data 11 as scatter plots, or the like, and provides a means for determining a health position or disease risk position from patterns where disease trends and the strength of health from the standard deviation are expressed as differences in red and blue density, and proximity to the mean is expressed using white (no color). If there is a positive correlation, for example, the number of generated replicas may be increased by a factor of one or two orders of magnitudes. Replicas may be generated in the form of adding increasing trends and conditions of what happens when numerical values have increased, and it has been confirmed that the matching accuracy increases when the number of samples has been increased in reality. When the number of sample data is limited, the number to be scaled may be selected at random and extrapolated while expanding at a certain ratio. Although there will be an error, the matching accuracy is much higher than when scaling is not performed. For example, it has been established that a line that calculates the mean of a population of 300 samples by extrapolating mean values obtained by randomly selecting 20, 40, 70, 100, or 130 will favorably match population samples that have been actually measured. Extrapolation is also effective for probability density and distribution trends. However, this is limited to data with clear correlation.

A module 1921 performs scaling processing (for disease) for heat map correlation 194, and in particular negative correlation. Processing for negative correlation is the same as for positive correlation, and when scaling is performed, extrapolation can be performed by the same method, and makes it possible to improve the prediction accuracy.

A module 1922 performs mixing of heat map correlation 194. When scaling and executing generation of replicas for the input data/test data 11 using the heat maps, mixing and hybridization may be performed while referring to the incidence and/or appearance frequency of diseases.

A module 1930 performs scaling (for diseases) of the vector map correlations (vector map) 195, and in particular scaling of positive correlation. The vector map 195 is a diagram (map) in which past input data/test data (deviation values) is viewed as changes over time and change amounts are expressed as vectors toward disease risk, while considering the autonomic nerve level and/or the immune system level based on fatigue, stress, sleep, exercise, and the like (it is also possible to determine the degree of immune system activity by directly examining leukocytes/NK cells, B cells, T cells, and macrophages). If there is any positive correlation in the disease risk and time direction in the vector map 195, the module 1930 expands the vector map with consideration to such correlation. It is possible to search for vectors that match the input data/test data (deviation values) of the subject, to estimate what dangers there are of heading from the current state to certain disease risks, and to increase the number of factors to be expanded.

A module 1931 performs scaling (for diseases) of the vector map correlations 195, and in particular of negative correlation. When the vector map 195 has negative correlations, this indicates that there is strong correlation toward a healthy state. Here, it is possible to increase the number of factors to be expanded through predictive estimation of whether the subject is recovering and returning to a healthy state.

A module 1932 performs mixing and hybridization of the vector map correlations 195. When the generation of replicas of the input data/test data is scaled using the vector map 195, a process of mixing and hybridization be performed while referring to the incidence and appearance frequency of diseases.

A module 1950 may expand the replica data to generate input replicas Xn corresponding to the disease risk, internal replicas corresponding to the disease risk, and regeneration replicas Rn corresponding to the disease risk, in a range where constraints obtained by the heat map 194 and the vector map 195 are not removed. The module 1960 outputs replica data 13 that serves as learning data. The module 1960 may output the actual measurement data A, the measurement replicas (input replicas) X, the internal replicas I, and the regeneration replicas R.

5. Cloud Service (P-HARP: Precision Health Analysis Research Platform)

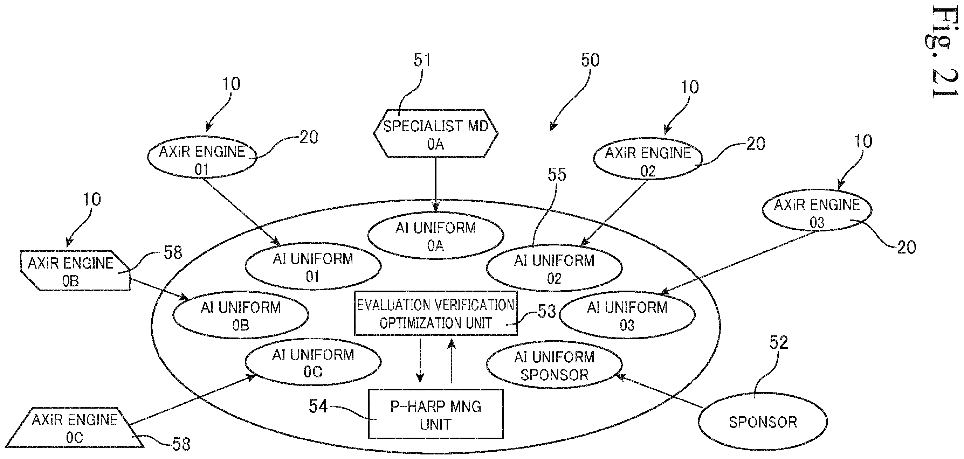

FIG. 21 depicts an overview of a cloud service (P-HARP) 50 provided by the present applicant. P-HARP 50 is a cloud-based platform that enables a decision-making team to be formed by one or more diagnosis support systems 10 that include the diagnosis support engine (AXiR engine) 20 that learns the input data and correlation of breast cancer, pancreatic cancer, kidney cancer (renal failure), and other diseases, and a specialist 51. In the P-HARP50, an AI suit or AI shell called the "AI uniform" 55 is provided as a common interface and API that link to other members such as other AIs 58 and/or applications to enable communication performed freely or with appropriate restrictions.

The P-HARP 50 includes a plurality of diagnosis support engines 20 a health care specialist (medical doctor or MD) 51, a sponsor 52, an evaluation verification optimization unit 53, and a management unit 54, and is a cloud-based global platform with high expandability where constituent members are connected via a computer network 59 such as an Intranet or the Internet. At the P-HARP 50, to facilitate the connecting of a plurality of AI engines 58 in the same or different specialized fields, the diagnosis engine 20 and other constituent members are connected through a shared interface called an AI uniform (or "AI shell" or "AI suit") 55.

As one example, when a member (sponsor) 52 such as a diabetic patient or a person who is worried about kidney function registers as a member of P-HARP 50 and enters his/her blood glucose level/ HbAlc or creatine level, the inputting of a medical interview, blood data, and/or urine data is requested. If there is a request from a member 52, information on analysis relating to the member 52 is provided. In addition, regarding health care information and services for maintaining and improving health, it is possible to share advice information for categories of interest to the member.

By providing a health care information service for a certain period of time to paid members (sponsors, members) 52, it is possible to give information and advice about whether such individuals are heading toward a favorable state of health or conversely are heading in a bad direction. However, as a general rule, since there is always a problem that it is not possible to guarantee a subject is in healthy condition due to false positives and false negatives, advice that requests a consultation with a specialist or a hospital may be finally given.

The P-HARP 50 may have locality. In this case, personal information may be excluded to the greatest degree possible (it may be unnecessary to specify individuals using login names). By having subjects enter their main area of activity (living sphere, residential area, workplace, or school), it is possible to grasp the spread of infectious diseases, influenza, and other problems. Accordingly, by using the P-HARP 50, it is possible to predict the spread of epidemics and infectious diseases and take early countermeasures. That is, by connecting a plurality of members 52, the diagnosis support engines 20 and the like via the P-HARP 50, it is possible to grasp and predict the state and spread of an infection in real time across society. In other words, the P-HARP 50 can provide a system where information on single individuals functions to protect society as a whole.

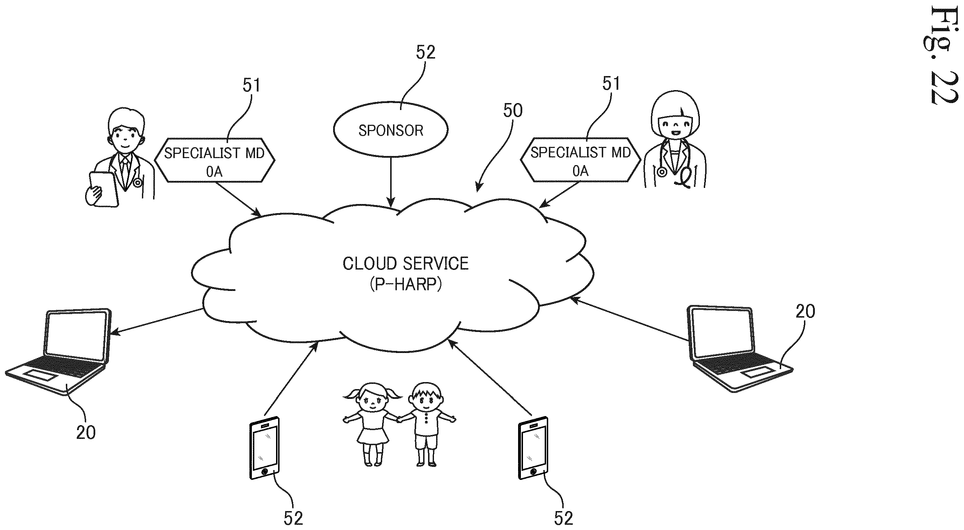

As depicted in FIG. 22, the P-HARP 50 may further include a function as a stadium that provides a place for collaborative work in which a plurality of diagnosis support engines 20 make inferences from different viewpoints. In the case of human society, people such as patients and defendants exist at general hospitals and courthouses, and specialists, medical staff, medical equipment, lawyers, prosecutors, judges, and magistrates (police) participate to solve their problems. The P-HARP 50 has a concept that resembles a place where treatment and sentencing are decided and executed, and may function as a stadium for making a diagnosis for a subject of diagnosis with higher accuracy through using a large number of participants. In other words, it is possible to realize an arrangement where a plurality of diagnosis engines 20 and a specialized medical doctor 51 who are assigned roles participate in the P-HARP 50 to increase the prediction accuracy and estimation accuracy.

The P-HARP 50 provides an interface between different AI as an AI protocol, and also provides an AI gateway as a function for realizing an interface with a human. Each member in the real world (RealWorld) is capable of mapping his/her health care information to an avatar in virtual reality (VR) on a network (Internet or Intranet). When doing so, it is possible to freely assume and use links to the real world. A team of specialized medical doctors makes predictions of health level and disease risk for avatars of individuals and groups based on collaborators who are registered in advance and real-world healthcare data. Tracing and additional tests and checks are also conducted if desired by individuals or groups.

The member 52 may be a sponsor company, aside from an individual or an organization who receives the service. The sponsor company may provide items in VR, may also do so as required or desired in the real world, may analyze the actual test data and the like collected by the P-HARP50 and publish the results as reference values, may search for new business by analyzing the accumulated big data, and/or may operate a business that provides additional information to other members 52. This means that there can be cases where members 52 connected to the P-HARP 50 may consent to the use of personal data in exchange for an agreed incentive.

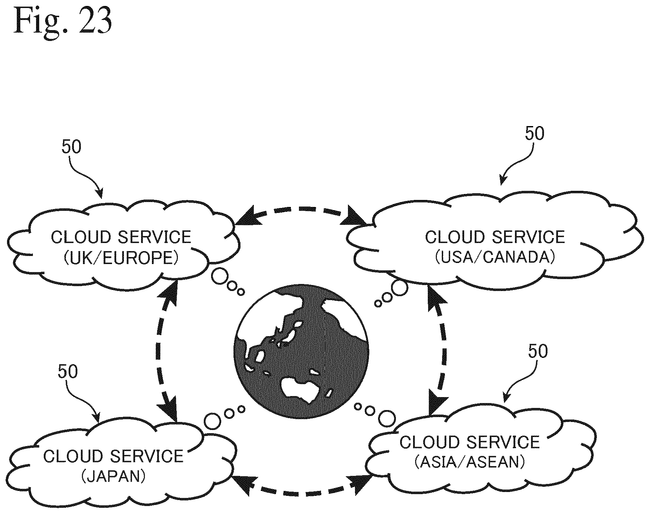

As depicted in FIG. 23, the P-HARP 50 may provide a high-precision measurement healthcare analysis research platform that is usable worldwide and enables a plurality of specialized diagnosis support engines 20 and experts (doctors and researchers) 51, engineers, experts in the field, and the like to exchange opinions and information via the cloud to promote analysis of big data and improve the overall prediction accuracy. The verification engine 23 of a diagnosis support engine 20 is capable of checking judgments made by a medical doctor 51 via the P-HARP 50. In a case where the diagnosis result is marginal, for example, when, the subject is in a presymptomatic state and there is slow development before the onset of a disease, with a normal method (prediction based on one set of measurement input data), it is expected to be difficult for the verification engine 23 to verify the result of predictive estimation. In this case, the verification engine 23 can request a doctor, a hospital, or the like connected to the P-HARP 50 to perform a follow-up observation via the P-HARP 50. The verification engine 23 may perform additional tests according to a procedure provided in advance via the P-HARP 50.

The P-HARP 50, the diagnosis support engine 20, and the diagnosis support system 10 can be connected to each other and are symmetrical in terms of communication. As one example, in the sense that multiple N: M connections are possible, there is no concept of higher order and lower order nodes. As depicted in FIG. 23, there may be a plurality of P-HARPs 50, which may be grouped by region, country, boundaries for preventing infectious diseases, or other units, or may have unique coverage. One P-HARP 50 may connect a plurality of diagnosis support engines 20 or diagnosis support systems 10, and may include human experts 51. The plurality of diagnosis support engines 20 to be connected may be engines of different inference capability, with different specialties and different viewpoints due to the use of different input data and learning. For the P-HARP 50, the number of diagnosis support engines 20 to be connected may be scaled according to the hardware scale.

It is also possible to form the P-HARP 50 in units of sponsors. To prevent limitations to the business model, it is possible to use a configuration that allows the maximum number of structures for design freedom and business implementation by individual sponsors. As one example, the issuing of tickets, user recruitment, and operation method are determined by a business model conceived by the owner who owns and manages the system. In principle, the interface conditions can be established based on the Internet connecting method called "TCP/IP protocol". However, a method that follows an AI protocol and AI gateway provided by Axion Research is adopted. In the future, by utilizing hardware security, there is the possibility that more advanced and widely applicable services will be offered through owners and managers.

That is, the name P-HARP 50 is an abbreviation for "P-Health Analysis Research Platform" and refers to a platform developed for health care purposes. The P-HARP 50 provides a space where the diagnosis support engines 20 (diagnosis support systems 10) called "AXiR Engines", other AI, experts, users, partners, members, and sponsors can be connected to each other and also provides services to perform analysis of and make predictions and inferences about the current status, activity level, disorder level, defects, abnormalities, emergencies, runaway situations, and the like of complex systems. The expression "P-HARP" 50 refers to a system or platform that has a premise of control by its owner or administrator, exists on the Internet, uses expertise and empirical rules, and allows the diagnosis support engines 20 and other actors to connect to each other through applications that are allow to connect and perform communication. A function for connecting to another P-HARP 50 is also implemented. The P-HARP 50 can use Watson and other AI on the outside by using natural language processing, voice processing, a gateway called an "AI uniform" (or "AI shell" or "AI suit"), or the like.

In the above description, there is disclosed a hybrid system 20 including an inference engine 22 for diagnosing a first subject (first object, first target system) of diagnosis, an artificial intelligence 21 provided in parallel with the inference engine, and an education system 30 that generates replicas of the input data 11 for educating the artificial intelligence, with respect to the diagnosis result of the inference engine and the diagnosis result of the artificial intelligence for the input data 11. The hybrid system 20 may include a final determination/deciding module 28 which gives priority, immediately after the start of education (learning), to the diagnosis result of the inference engine 22 as the diagnosis result or diagnosis support result of the hybrid system 20, and is operable when the accuracy of the diagnosis result of the artificial intelligence 21 exceeds the accuracy of the diagnosis result of the inference engine 22, to output the diagnosis result of the artificial intelligence 21 with priority as the diagnosis result of the hybrid system 20. The education system 30 may include: a verification engine 23 that verifies the diagnosis result of the inference engine 22 and the diagnosis result of the artificial intelligence 21; an optimization engine 24 that generates an optimization policy for replicas of the input data based on the verification results of the verification engine; and a replica generating module 19 that generates replicas 13 of the input data 11 based on the input data 11 and instructions from the optimization engine 24.

The replica generating module 19 may generate first replicas of the input data; second replicas indicating the states of the first subject of diagnosis; and third replicas that serve as replicas of input data reversely estimated from the range of change of the second replicas. The verification engine 23 may include a function that requests, when the verification engine 23 determines that it is difficult to verify the diagnosis result of the inference engine 22 and the diagnosis result of the artificial intelligence 21, advice of a diagnosis expert and/or a follow-up observation of the first subject of diagnosis. An output module 29 that outputs the diagnosis result of the inference engine 22 or the diagnosis result of the artificial intelligence 21 as the state of a digital avatar may also be provided.

There is also disclosed a cloud system 50 in which at least one hybrid system 20 and a terminal of a diagnosis expert on the first subject of diagnosis are connected via the cloud, and a diagnosis result of the at least one hybrid system is referred to via the cloud. In the cloud system 50, the terminal 51 of a diagnosis expert on the first subject of diagnosis and an institution 52 capable of a follow-up observation of the first subject of diagnosis are connected by a cloud, and a verification engine 23 of the at least one hybrid system may order advice from a diagnosis expert and/or a follow-up observation via the cloud.

The first feature of the above configuration is to implement a mechanism operable when there is a serious discovery related to human life, to store this discovery with the highest priority. Here, a "fatal event" covers both external factors and internal factors. If there is a fatal discovery for the system, a mechanism that gives this top priority is added. In the case of healthcare, a function that shares and gives warning about events and signs of cancer, infections, and events that make it difficult to maintain life and a favorable state of health, investigates causes and correlations, and accumulates such information in the diagnosis engine 20 and the P-HARP 50 to promote sharing may be implemented. Sharing and giving warnings for events that make it difficult to maintain a system or maintain an individual is incorporated because it is an important function for a complex system.

The second feature of the above configuration is about situations (such as events or sudden changes over time) that require an emergency response. When a situation requiring emergency response occurs during 24-hour monitoring (continuous measurement), the phenomena occurred before and after, and in particular data that needs to be analyzed even if it may be unknown at the time, are stored and subsequently used for data analysis. Also, for the same reasons as the first feature given above, when there is high urgency, a process that initializes a procedure that minimizes the damage is implemented. This goes beyond a monitoring function, and in more detail includes actions that involve system shutdowns and emergency procedures.

The third feature of the above configuration is self- optimization (self-learning). The optimization engine 24 controls the replica generator 19 so as to increase the inference (prediction) accuracy of the AI engine 21 from the result of the verification engine 23. Adjustment progresses so that the difference (error) between the evaluation function in the meta model description file 14 and the verification data is reduced. In the case of the AI engine 21, learning proceeds from actual measurement data and additional detailed test results (inputs), but learning can also be accelerated using medical interview data and replica data.

The fourth feature of the above configuration is the idea or viewpoint of using external AI. External AI includes speech recognition technology, document and text creation technology, academic paper analysis technology, a conversation (interpreting) function, and the like, and includes an AI shell interface and AI protocol that enables the results of external AI, such as Watson, to be used. When a human expert is included, this is covered by the AI gateway.

The fifth feature of the above configuration is switching between the inference engine 22 and the AI engine 21. A mechanism for switching between these two and self-learning is important as a mechanism that can educate the AI engine 21 in a short time. This switching may be reversed later.

The sixth feature of the above configuration is to prevent a runaway situation (abnormality) for an OPEN/CLOSE system. This system includes a hot line to the inside like a VPN, so that control and access from the outside are possible when the system is running in the OPEN state and running in the CLOSE state. The diagnosis engine 20 and the AI suit implement a timer/hardware security-ring method authentication tracing software that guarantees normal operation, and the Primary Master and Secondary Master periodically check profiles according to an AI protocol determined for each member situation. If this activity and contribution/reaction are not confirmed for a certain period of time, all security/rings are blocked off to make access to the inside impossible. When moving a digital avatar to another VR cyberspace, another key needs to be exchanged to execute the movement, and while there is a degree of freedom for movement, a trace will remain.

The seventh feature of the above configuration is to infer the determination process by analogy from past trends and future trends. This includes dynamically inference by analogy through dialogue with experts. The verification engine 23 has a function of performing (ordering) additional tests and follow-up observations as handling of a verifiable cases and pending cases. From the viewpoint seeing how gray zone subjects and pending subjects change in units of one to two weeks using changes over time and a method involving detailed examination of additional tests, it is possible, by conducting detailed examination of additional tests and repeating this several times, to reduce the number of unclear subjects in the entire population.

In the above description, a target system for diagnosis support has been described with the human body as an example. The target of diagnosis support is not limited to the human body, but may be another system, and in particular a complex system including many elements, mechatronics, such as plant or an engine, or a service such as finance or investment. The system may also be a game or system with other big data.

**Claims**

1. A support system for estimating an internal state of a target system, comprising a set of modules, wherein the set of modules includes:

   an inference module configured to output a first estimated state of the target system that has been estimated according to at least one predetermined rule, based on first test data relating to the target system;

an artificial intelligence module that includes a learning module that is configured to perform learning to estimate a state of the target system based on the first test data and the first estimated state, and is configured to output a second estimated state of the target system for second test data;

a verification module configured to verify accuracy of state estimated by the inference module and state estimated by the artificial intelligence module; and

a determination module that switches priority rankings between the state estimated by the inference module and the state estimated by the artificial intelligence module to output, based on a verification result of the verification module.

2. The support system according to claim 1, wherein the set of modules further includes a replica generating module configured to generate replicas of test data of the target system for learning by the learning module.

3. The support system according to claim 2, wherein the set of modules further includes an optimization module configured to provide an optimization policy for the replicas to the replica generating module based on accuracy of the state estimated by the artificial intelligence module that has been verified by the verification module.

4. The support system according to claim 2 or 3, wherein the replica generating module includes a module that generates the replicas that reflect actual distributions of a plurality of parameter values included in test data of the target system.

5. The support system according to any one of claims 2 to 4, wherein the replica generating module includes a module that generates internal replicas indicating internal states of the target system and replicas of test data reversely estimated from ranges of change in the internal replicas.

6. The support system according to any one of claims 1 to 5, wherein the set of modules further includes an input processing module configured to pre-process test data of the target system according to a predetermined rule to convert to test data to be inputted into the inference module and the artificial intelligence module.

7. The support system according to claim 6, wherein the input processing module includes a module that generates a map indicating correlations between parameters included in the test data.

8. The support system according to any one of claims 1 to 7, wherein the verification module includes:

   a module for determining ambiguity in the state estimated by the inference module or the artificial intelligence module; and
   a module that acquires additional information from outside in accordance with the ambiguity.

9. The support system according to claim 8, wherein the additional information includes at least one of feedback from a diagnosis expert on the target system, a follow-up observation, and a result of an additional test.

10. The support system according to any one of claims 1 to 9, wherein the set of modules further includes a module that outputs the estimated state selected by the determination module as diagnosis information on the target system.

11. The support system according to any one of claims 1 to 10, wherein the target system is a human body.

12. The support system according to any one of claims 1 to 11, further comprising:

    a memory storing the set of modules as a program; and
    a processor that loads and executes the program stored in the memory.

13. A cloud system, where at least one support system according to any one of claims 1 to 12 and a terminal of a diagnosis expert on the target system are connected by a cloud, and an output of the at least one support system is referenced via the cloud.

14. A cloud system, where at least one support system according to claim 9, a terminal of a diagnosis expert on the target system, and an institution capable of a follow-up observation of the target system are connected by a cloud, and the verification module of the at least one support system orders advice of the diagnosis expert or the follow-up observation via the cloud.

15. A program comprising instructions that cause a computer to function as a support system according to any one of claims 1 to 12.

16. A control method for a support system that estimates an internal state of a target system, wherein the support system includes:

an inference module configured to output a first estimated state of the target system that has been estimated according to at least one predetermined rule, based on first test data relating to the target system;

an artificial intelligence module that includes a learning module that is configured to perform learning to estimate a state of the target system based on the first test data and the first estimated state, and is configured to output a second estimated state of the target system for second test data; and

a verification module configured to verify accuracy of state estimated by the inference module and state estimated by the artificial intelligence module, and

the control method comprises:

outputting the state estimated by the inference module with priority over the state estimated by the artificial intelligence module; and

outputting the state estimated by the artificial intelligence module with priority over the state estimated by the inference module.

17. The control method according to claim 16, wherein the support system further includes a replica generating module configured to generate replicas of test data of the target system for learning by the learning module, and

the control method further comprises providing an optimization policy for the replicas to the replica generating module based on accuracy of the state estimated by the artificial intelligence module that has been verified by the verification module.

# Fig. 1

# Fig. 2

Fig. 3

Fig. 4

Fig. 5

Fig. 6

Fig. 7

(a) 131     (b) 132     (c) 133

Fig. 8

# Fig. 9

Fig. 10

(a)

SN LDL vs TC

131
139

TC

240
220
200
180
160

80  100  120  140  160
LDL

(b)

ND LDL vs TC

132
139

TC

260
240
220
200
180
160

100  150
LDL

(c)

DS LDL vs TC

133
139

TC

400
350
300
250
200
150
100

100  200  300
LDL

# Fig. 11

(a)

LDL

137
136
135

(b)

TC

137
136
135

Fig. 12

Fig. 13

EP 3 660 752 A1

BASIC SYSTEM — 40

10   20(110)

11 — TEST DATA (INPUT DATA)

15   30

INPUT/OUTPUT CORRELATION TABLE DESCRIPTION FILE

AI LEARNING SYSTEM

MAP STORED AND REFERRED TO

27 — STATISTICAL CORRELATION PROCESSING MODULE

GENERATE DATA/SEMI-AUTOMATIC OR MANUAL

14 — META MODEL DESCRIPTION FILE

22   21

DATA INPUT   DATA INPUT

INFERENCE ENGINE   AI ENGINE

LEARN

REFER

LEARNING DATA (TEACHING DATA) — 35

19

OUTPUT DETERMINATION RESULT FOR PRESYMPTOMATIC DISEASE

OUTPUT DETERMINATION RESULT FOR PRESYMPTOMATIC DISEASE

GENERATE DATA/SEMI-AUTOMATIC OR MANUAL

13 — REPLICA DATA

REPLICA GENERATING MODULE

REFER

STORE OF ACCURACY OF ENGINES

23

OUTPUT VERIFICATION RESULTS

VERIFICATION ENGINE

GENERATE DATA

CONTROL TREND OF DATA

OPTIMIZATION ENGINE

16

OUTPUT RESULT OF INFERENCE ENGINE AND RESULT OF AI ENGINE

28

REFER

24

REFER

ENGINE ACCURACY DATABASE

REFER

FINAL DETERMINATION/DECIDING MODULE

12

OUTPUT RESULT WITH BEST PREDICTION ACCURACY

OUTPUT DATA

45

Fig. 14

# Fig. 15

45

20

MEDICAL DOCTOR

REQUEST ADDITIONAL TESTS

ADDITIONAL DATA

PREDICTION RESULT OF HEALTH STATE

PREDICTION RESULT OF HEALTH STATE

ADJUSTMENT OF AI ENGINE

ADJUSTMENT OF REPLICA GENERATION METHOD

VERIFICATION ENGINE — 23

EVALUATION MODEL — 23M

PREDICTION RESULT — 12

INFERENCE ENGINE — 22

AI ENGINE — 21

REPLICAS

REPLICA GENERATING MODULE — 19

INPUT DATA (INVASIVE, NONINVASIVE, QUESTIONNAIRE, OTHERS) — 11

ACTUAL DATA

# Fig. 16

Fig. 17

WHERE THERE IS LITTLE DATA

INPUT DATA — 11

INFERENCE ENGINE — 22

AI ENGINE — 21

OUTPUT RESULT — 12

— 20

INCREASE NUMBER OF DATA

WHERE THERE IS LOTS OF DATA

INPUT DATA — 11

INFERENCE ENGINE — 22

AI ENGINE — 21

OUTPUT RESULT — 12

— 20

EP 3 660 752 A1

Fig. 18

Fig. 19

290

OUTPUT INFORMATION
TABLE

28

FINAL DETERMINATION/
DECIDING MODULE

PREDICTION
RESULT

29

OUTPUT PROCESSING
MODULE

REFER

INDEX
INFORMATION

REFER

P-HARP

50

# Fig. 20

REPLICAS A, X, I, R
LEARNING DATA ~ 13

OPTIMIZATION ENGINE
PREDICTION/INFERENCE
ACCURACY UP ~ 24

REPLICA CONTROL FILE
REPLICA GENERATION CONTROL ~ 191

MODEL FILE X, I, R REPLICA GENERATION
PROBABILITY DENSITY ~ 192

REPLICA FILE A, X, I REPLICAS ~ 193

REPLICA GENERATING MODULE ~ 19

20

SAMPLE PROCESSING
STATISTICAL FEATURE EXTRACTION ~ 1910

MIXED NORMAL DISTRIBUTION
ANALYSIS
1~N SUPERPOSITION ~ 1911

VARIANCE/PROBABILITY DENSITY,
CORRELATIONS, AND CONSTRAINT
CONDITIONS ~ 1912

REPLICA DATA
X, I, R, Gr. ~ 1915

REPLICA DATA EXPANSION
Xn, In, Rn/DISEASE ~ 1950

EPLICA DATA OUTPUT
PROCESSING ~ 1960

INPUT DATA A TEST DATA/
MEDICAL INTERVIEW ~ 11

HEAT MAP
HEALTHY, PRESYMPTOMATIC,
DISEASED ~ 194

HEAT MAP CORRELATION
POSITIVE CORRELATION > SCALING ~ 1920

HEAT MAP CORRELATION
NEGATIVE CORRELATION > SCALING ~ 1921

HEAT MAP CORRELATION
MIXING ~ 1922

VECTOR MAP
HEALTHY, PRESYMPTOMATIC,
DISEASED ~ 195

VECTOR MAP CORRELATION
POSITIVE CORRELATION > SCALING ~ 1930

VECTOR MAP CORRELATION
NEGATIVE CORRELATION > SCALING ~ 1931

HEAT MAP CORRELATION
MIXING ~ 1932

Fig. 21

Fig. 22

Fig. 23

# EP 3 660 752 A1

International application No.

PCT/JP2018/027735

| A. CLASSIFICATION OF SUBJECT MATTER |
|---|
| Int.Cl. G06N99/00(2010.01)i, G06Q50/22(2018.01)i |

According to International Patent Classification (IPC) or to both national classification and IPC

| B. FIELDS SEARCHED |
|---|
| Minimum documentation searched (classification system followed by classification symbols) |
| Int.Cl. G06N99/00, G06Q50/22 |

| Documentation searched other than minimum documentation to the extent that such documents are included in the fields searched | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| X<br>Y<br>A | JP 2002-133390 A (MATSUSHITA ELECTRIC INDUSTRIAL CO., LTD.) 10 May 2002, paragraphs [0032]-[0153], fig. 1A-11 (Family: none) | 1-2, 6-7, 10-12, 15-16<br>8-9, 13-14<br>3-5, 17 |
| Y | JP 2013-165780 A (CANON INC.) 29 August 2013, paragraphs [0054]-[0069], fig. 3-5 & US 2015/0006447 A1, paragraphs [0064]-[0078], fig. 3-5 & CN 104114083 A & KR 10-2014-0131346 A | 8-9, 13-14 |

☐ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 27 August 2018 (27.08.2018) | 04 September 2018 (04.09.2018) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**EP 3 660 752 A1**

**Patent documents cited in the description**

- JP 2017091586 A